# EUROPÄISCHE PATENTSCHRIFT

(11) **EP 0 946 544 B1**
(45) Veröffentlichungstag und Bekanntmachung des Hinweises auf die Patenterteilung: **28.04.2004**
(21) Anmeldenummer: 97954724.7
(22) Anmeldetag: 05.12.1997
(51) Int. Cl.: C07D 401/04, A01N 43/54

(54) **HETEROCYCLYLURACILE**
HETEROCYCLYLURACIL
HETEROCYCLYLURACILES

(30) Priorität: 17.12.1996 DE 19652429
(43) Veröffentlichungstag der Anmeldung: 06.10.1999
(73) Patentinhaber: Bayer CropScience AG, 40789 Monheim (DE)
(72) Erfinder: ANDREE, Roland, D-40764 Langenfeld (DE); DREWES, Mark, Wilhelm, D-40764 Langenfeld (DE); HAAS, Wilhelm, D-50259 Pulheim (DE); DOLLINGER, Markus, D-51381 Leverkusen (DE)
(86) Internationale Anmeldenummer: PCT/EP1997/006819
(87) Internationale Veröffentlichungsnummer: WO 1998/027082

(56) Entgegenhaltungen:
- CHEMICAL ABSTRACTS, vol. 120, no. 9, 1994 Columbus, Ohio, US; abstract no. 107048v, Seite 1170; XP002064551 in der Anmeldung erwähnt & JP 05 202 031 A (NISSAN) 10.August 1993
- CHEMICAL ABSTRACTS, vol. 116, no. 28, 1992 Columbus, Ohio, US; abstract no. 235650q, Seite 833; XP002064552 in der Anmeldung erwähnt & JP 03 287 585 A (NISSAN) 18.Dezember 1991

## Beschreibung

Die vorliegende Erfindung betrifft neue Heterocyclyluracile, mehrere Verfahren zu ihrer Herstellung und ihre Verwendung als Herbizide.

Es sind bereits zahlreiche Heterocyclyluracile mit herbiziden bzw. insektiziden Eigenschaften bekannt geworden (vgl. JP-A 91-287 585, JP-A 93 202 031, Chem. Abstr. 116, 235 650 und Chem. Abstr. 120, 107 048). So läßt sich z.B. 1-(3-Chlor-5-trifluoromethylpyridin-2-yl)-3,6-dihydro-2,6-dioxo-4-trifluormethyl-1(2H)-pyrimidin zur Bekämpfung von Unkraut verwenden. Bei niedrigen Aufwandmengen ist die Wirksamkeit dieses Stoffes aber nicht immer befriedigend.

Es wurden nun neue Heterocyclyluracile der Formel 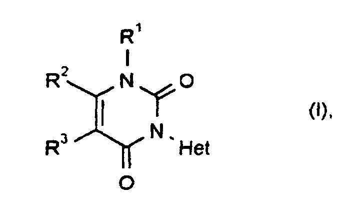 in welcher
- R¹: für Wasserstoff oder für gegebenenfalls durch Cyano, Halogen oder C₁-C₄-Alkoxy substituiertes C₁-C₄-Alkyl steht,
- R²: für Formyl, Hydroximinomethyl, Cyano, Carboxy, Alkoxycarbonyl, Carbamoyl, Thiocarbamoyl® oder für gegebenenfalls durch Halogen substituiertes C₁-C₄-Alkyl steht,
- R³: für Wasserstoff, Cyano, Halogen oder für gegebenenfalls durch Halogen substituiertes C₁-C₄-Alkyl steht und
- Het: für den Rest der Formel 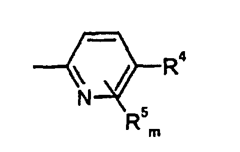 steht, worin
- R⁴: für Cyano oder Thiocarbamoyl steht,
- R⁵: für Alkoxy mit 1 bis 6 Kohlenstoffatomen, Halogenalkoxy mit 1 bis 6 Kohlenstoffatomen und 1 bis 5 Halogenatomen, Alkoxyalkoxy mit 1 bis 6 Kohlenstoffatomen in jedem Alkoxyteil, Alkylthio mit 1 bis 6 Kohlenstoffatomen, Halogenalkylthio mit 1 bis 6 Kohlenstoffatomen und 1 bis 5 Halogenatomen, Alkylsulfonylamino mit 1 bis 6 Kohlenstoffatomen im Alkylteil, Halogenalkylsulfonylamino mit 1 bis 5 Halogenatomen und 1 bis 6 Kohlenstoffatomen im Halogenalkylteil, N,N-Bis-alkylsulfonylamino mit 1 bis 6 Kohlenstoffatomen in jedem Alkylteil, N,N-Bis-Halogenalkylsulfonyl-amino mit 1 bis 5 Halogenatomen und 1 bis 6 Kohlenstoffatomen in jedem Halogenalkylteil, N-Alkyl-N-alkylsulfonyl-amino mit 1 bis 6 Kohlenstoffatomen im Alkylteil und 1 bis 6 Kohlenstoffatomen im Alkylsulfonylteil, N-Halogenalkyl-N-halogenalkylsulfonyl-amino mit 1 bis 5 Halogenatomen und 1 bis 6 Kohlenstoffatomen im Halogenalkylteil und 1 bis 5 Halogenatomen und 1 bis 6 Kohlenstoffatomen im Halogenalkylsulfonylteil, N-Alkylcarbonyl-N-alkylsulfonyl-amino mit 1 bis 6 Kohlenstoffatomen im Alkylteil der Alkylcarbonylgruppe und 1 bis 6 Kohlenstoffatomen im Alkylsulfonylteil, N-Halogenalkylcarbonyl-N-halogenalkylsulfonyl-amino mit 1 bis 5 Halogenatomen und 1 bis 6 Kohlenstoffatomen im Halogenalkylteil und 1 bis 5 Halogenatomen und 1 bis 6 Kohlenstoffatomen im Halogenalkylsulfonylteil oder für gegebenenfalls einfach bis dreifach, gleichartig oder verschieden durch Halogen, Alkyl mit 1 bis 4 Kohlenstoffatomen, Dialkylamino mit 1 bis 4 Kohlenstoffatomen in jeder Alkylgruppe und/oder Alkoxy mit 1 bis 4 Kohlenstoffatomen substituiertes N-Alkylsulfonyl-N-phenylcarbonyl-amino mit 1 bis 6 Kohlenstoffatomen im Alkylsulfonylteil steht, und
- m: für ganze Zahlen von 0 bis 3 steht,
oder
- Het: für den Rest der Formel 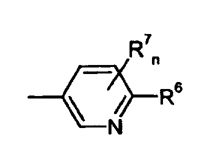 steht, worin
- R⁶: für Carbamoyl oder Thiocarbamoyl steht,
- R⁷: für Alkoxy mit 1 bis 6 Kohlenstoffatomen, Halogenalkoxy mit 1 bis 6 Kohlenstoffatomen und 1 bis 5 Halogenatomen, Alkoxyalkoxy mit 1 bis 6 Kohlenstoffatomen in jedem Alkoxyteil, Alkylthio mit 1 bis 6 Kohlenstoffatomen, Halogenalkylthio mit 1 bis 6 Kohlenstoffatomen und 1 bis 5 Halogenatomen, Alkylsulfonylamino mit 1 bis 6 Kohlenstoffatomen im Alkylteil, Halogenalkylsulfonylamino mit 1 bis 5 Halogenatomen und 1 bis 6 Kohlenstoffatomen im Halogenalkylteil, N,N-Bis-alkylsulfonylamino mit 1 bis 6 Kohlenstoffatomen in jedem Alkylteil, N,N-Bis-Halogenalkylsulfonyl-amino mit 1 bis 5 Halogenatomen und 1 bis 6 Kohlenstoffatomen in jedem Halogenalkylteil, N-Alkyl-N-alkylsulfonyl-amino mit 1 bis 6 Kohlenstoffatomen im Alkylteil und 1 bis 6 Kohlenstoffatomen im Alkylsulfonylteil, N-Halogenalkyl-N-halogenalkylsulfonyl-amino mit 1 bis 5 Halogenatomen und 1 bis 6 Kohlenstoffatomen im Halogenalkylteil und 1 bis 5 Halogenatomen und 1 bis 6 Kohlenstoffatomen im Halogenalkylsulfonylteil, N-Alkylcarbonyl-N-alkylsulfonyl-amino mit 1 bis 6 Kohlenstoffatomen im Alkylteil der Alkylcarbonylgruppe und 1 bis 6 Kohlenstoffatomen im Alkylsulfonylteil, N-Halogenalkylcarbonyl-N-halogenalkylsulfonyl-amino mit 1 bis 5 Halogenatomen und 1 bis 6 Kohlenstoffatomen im Halogenalkylteil und 1 bis 5 Halogenatomen und 1 bis 6 Kohlenstoffatomen im Halogenalkylsulfonylteil oder für gegebenenfalls einfach bis dreifach, gleichartig oder verschieden durch Halogen, Alkyl mit 1 bis 4 Kohlenstoffatomen, Dialkylamino mit 1 bis 4 Kohlenstoffatomen in jeder Alkylgruppe und/oder Alkoxy mit 1 bis 4 Kohlenstoffatomen substituiertes N-Alkylsulfonyl-N-phenylcarbonyl-amino mit 1 bis 6 Kohlenstoffatomen im Alkylsulfonylteil steht, und
- n: für ganze Zahlen von 0 bis 3 steht,
oder
- Het: für den Rest der Formel 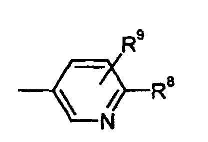 steht, worin
- R⁸: für Cyano steht und
- R⁹: für Alkylsulfonylamino mit 1 bis 6 Kohlenstoffatomen im Alkylteil, Halogenalkylsulfonylamino mit 1 bis 5 Halogenatomen und 1 bis 6 Kohlenstoffatomen im Halogenalkylteil, N,N-Bis-alkylsulfonylamino mit 1 bis 6 Kohlenstoffatomen in jedem Alkylteil, N,N-Bis-Halogenalkylsulfonyl-amino mit 1 bis 5 Halogenatomen und 1 bis 6 Kohlenstoffatomen in jedem Halogenalkylteil, N-Alkyl-N-alkylsulfonyl-amino mit 1 bis 6 Kohlenstoffatomen im Alkylteil und 1 bis 6 Kohlenstoffatomen im Alkylsulfonylteil, N-Halogenalkyl-N-halogenalkylsulfonyl-amino mit 1 bis 5 Halogenatomen und 1 bis 6 Kohlenstoffatomen im Halogenalkylteil und 1 bis 5 Halogenatomen und 1 bis 6 Kohlenstoffatomen im Halogenalkylsulfonylteil, N-Alkylcarbonyl-N-alkylsulfonyl-amino mit 1 bis 6 Kohlenstoffatomen im Alkylteil der Alkylcarbonylgruppe und 1 bis 6 Kohlenstoffatomen im Alkylsulfonylteil, N-Halogenalkylcarbonyl-N-halogenalkylsulfonyl-amino mit 1 bis 5 Halogenatomen und 1 bis 6 Kohlenstoffatomen im Halogenalkylteil und 1 bis 5 Halogenatomen und 1 bis 6 Kohlenstoffatomen im Halogenalkylsulfonylteil oder für gegebenenfalls einfach bis dreifach, gleichartig oder verschieden durch Halogen, Alkyl mit 1 bis 4 Kohlenstoffatomen, Dialkylamino mit 1 bis 4 Kohlenstoffatomen in jeder Alkylgruppe und/oder Alkoxy mit 1 bis 4 Kohlenstoffatomen substituiertes N-Alkylsulfonyl-N-phenylcarbonyl-amino mit 1 bis 6 Kohlenstoffatomen im Alkylsulfonylteil steht,
gefunden.

Weiterhin wurde gefunden, daß man Heterocyclyluracile der Formel (I) erhält, wenn man
a) Aminoalkensäureester der Formel 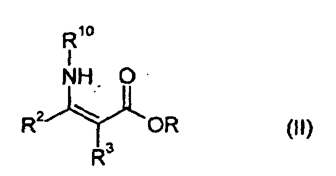 in welcher
   - R² und R³: die oben angegebenen Bedeutungen haben,
   - R¹⁰: für Wasserstoff oder Alkyl mit 1 bis 4 Kohlenstoffatomen steht und
   - R: für Alkyl mit 1 bis 4 Kohlenstoffatomen, Phenyl oder Benzyl steht,
   entweder
   α) mit Heterocyclylisocyanaten der Formel

      O=C=N-Het (III),

      in welcher
      - Het: die oben angegebene Bedeutung hat,
      oder
   β) mit Heterocyclylcarbamaten der Formel 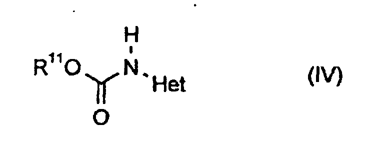 in welcher
      - Het: die oben angegebene Bedeutung hat und
      - R¹¹: für Alkyl mit 1 bis 4 Kohlenstoffatomen, Phenyl oder Benzyl steht,
      jeweils gegebenenfalls in Gegenwart eines Säurebindemittels und gegebenenfalls in Gegenwart eines Verdünnungsmittels umsetzt,
   oder
b) Heterocyclyluracile der Formel 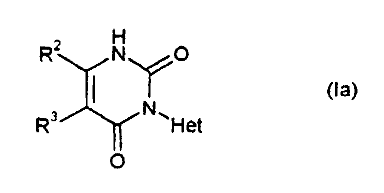 in welcher
   R², R³ und Het die oben angegebenen Bedeutungen haben,
   entweder
   α) mit Halogenverbindungen der Formel

      R¹²-Hal (V)

      in welcher
      - R¹²: für gegebenenfalls durch Cyano, Halogen oder Alkoxy mit 1 bis 4 Kohlenstoffatomen substituiertes Alkyl mit 1 bis 4 Kohlenstoffatomen steht und
      - Hal: für Chlor, Brom oder Iod steht,
      oder
   β) mit Dialkylsulfaten der Formel 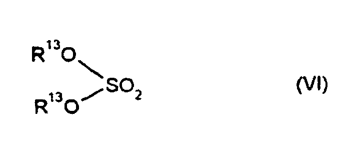
   in welcher
   - R¹³: für Alkyl mit 1 bis 4 Kohlenstoffatomen steht,
   jeweils gegebenenfalls in Gegenwart eines Säurebindemittels und gegebenenfalls in Gegenwart eines Verdünnungsmittels umsetzt.

Schließlich wurde gefunden, daß die neuen Heterocyclyluracile der Formel (I) sehr gute herbizide Eigenschaften besitzen.

Überraschenderweise zeigen die erfindungsgemäßen Heterocyclyluracile der Formel (I) eine wesentlich bessere herbizide Wirksamkeit als die konstitutionell ähnlichsten, vorbekannten Wirkstoffe gleicher Wirkungsrichtung.

Unter Alkyl, Alkoxy, Alkylthio, Alkylamino, Dialkylamino, Halogenalkyl, Halogenalkoxy und Halogenalkylthio sind im vorliegenden Fall jeweils geradkettige oder verzweigte Reste zu verstehen.

Halogen steht im vorliegenden Fall - wenn nicht anders angegeben - für Fluor, Chlor, Brom oder lod.

Die erfindungsgemäßen Heterocyclyluracile sind durch die Formel (I) allgemein definiert. Bevorzugt sind die Verbindungen der Formel (I), in denen
- R¹: für Wasserstoff oder für gegebenenfalls durch Cyano, Fluor, Chlor, Methoxy oder Ethoxy substituiertes Alkyl mit 1 bis 3 Kohlenstoffatomen steht,
- R²: für Formyl, Hydroximinomethyl, Cyano, Carboxy, Alkoxycarbonyl mit 1 bis 4 Kohlenstoffatomen im Alkoxyteil, Carbamoyl, Thiocarbamoyl oder für gegebenenfalls einfach bis dreifach, gleichartig oder verschieden durch Fluor und/oder Chlor substituiertes Alkyl mit 1 bis 3 Kohlenstoffatomen steht,
- R³: für Wasserstoff, Cyano, Fluor, Chlor oder für gegebenenfalls einfach bis dreifach, gleichartig oder verschieden durch Fluor und/oder Chlor substituiertes Alkyl mit 1 bis 3 Kohlenstoffatomen steht und
- Het: für den Rest der Formel 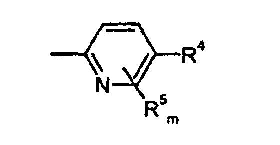 steht, worin
- R⁴: für Cyano oder Thiocarbamoyl steht,
- R⁵: für Alkoxy mit 1 bis 4 Kohlenstoffatomen, Halogenalkoxy mit 1 bis 3 Fluor- und/oder Chloratomen und 1 bis 4 Kohlenstoffatomen, Alkoxyalkoxy mit 1 bis 4 Kohlenstoffatomen in jedem Alkoxyteil, Alkylthio mit 1 bis 4 Kohlenstoffatomen, Halogenalkylthio mit 1 bis 3 Fluor- und/oder Chloratomen und 1 bis 4 Kohlenstoffatomen, Alkylsulfonylamino mit 1 bis 4 Kohlenstoffatomen, Halogenalkylsulfonylamino mit 1 bis 3 Fluor- und/oder Chloratomen und 1 bis 4 Kohlenstoffatomen, N,N-Bis-alkylsulfonylamino mit 1 bis 4 Kohlenstoffatomen in jedem Alkylteil, N,N-Bis-halogenalkylsul-fonyl-amino mit 1 bis 3 Fluorund/oder Chloratomen und 1 bis 4 Kohlenstoffatomen in jedem Halogenalkylteil, N-Alkyl-N-alkylsulfonyl-amino mit 1 bis 4 Kohlenstoffatomen im Alkylteil und 1 bis 4 Kohlenstoffatomen im Alkylsulfonylteil, N-Halogenalkyl-N-halogenalkylsulfonyl-amino mit 1 bis 3 Fluor- und/oder Chloratomen und 1 bis 4 Kohlenstoffatomen im Halogenalkylteil und mit 1 bis 3 Fluor- und/oder Chloratomen und 1 bis 4 Kohlenstoffatomen im Halogenalkylsulfonylteil, N-Alkylcarbonyl-N-alkylsulfonyl-amino mit 1 bis 4 Kohlenstoffatomen im Alkylteil der Alkylcarbonylgruppe und 1 bis 4 Kohlenstoffatomen im Alkylsulfonylteil, N-Halogenalkylcarbonyl-N-halogenalkylsulfonyl-amino mit 1 bis 3 Fluor- und/oder Chloratomen und 1 bis 4 Kohlenstoffatomen im Halogenalkylteil der Halogenalkylcarbonylgruppe und mit 1 bis 3 Fluor- und/oder Chloratomen und 1 bis 4 Kohlenstoffatomen im Halogenalkylsulfonylteil oder für gegebenenfalls einfach bis dreifach, gleichartig oder verschieden durch Fluor, Chlor, Brom, Methyl, Ethyl, n-Propyl, i-Propyl, n-Butyl, i-Butyl, s-Butyl, t-Butyl, Dimenthylamino, Diethylamino, Methoxy, Ethoxy, n-Propoxy und/oder i-Propoxy substituiertes N-Alkylsulfonyl-N-phenylcarbonyl-amino mit 1 bis 4 Kohlenstoffatomen im Alkylsulfonylteil steht, und
- m: für die Zahlen 0, 1 oder 2 steht, wobei R⁵ für gleiche oder verschiedene Reste steht, wenn m für 2 steht,
oder
- Het: für den Rest der Formel 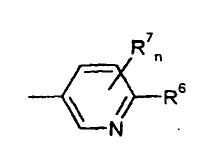 steht, worin
- R⁶: für Carbamoyl oder Thiocarbamoyl steht,
- R⁷: für Alkoxy mit 1 bis 4 Kohlenstoffatomen, Halogenalkoxy mit 1 bis 3 Fluor- und/oder Chloratomen und 1 bis 4 Kohlenstoffatomen, Alkoxyalkoxy mit 1 bis 4 Kohlenstoffatomen in jedem Alkoxyteil, Alkylthio mit 1 bis 4 Kohlenstoffatomen, Halogenalkylthio mit 1 bis 3 Fluorund/oder Chloratomen und 1 bis 4 Kohlenstoffatomen, Alkylsulfonylamino mit 1 bis 4 Kohlenstoffatomen, Halogenalkylsulfonylamino mit 1 bis 3 Fluor- und/oder Chloratomen und 1 bis 4 Kohlenstoffatomen, N,N-Bis-alkylsulfonylamino mit 1 bis 4 Kohlenstoffatomen in jedem Alkylteil, N,N-Bis-halogenalkylsulfonyl-amino mit 1 bis 3 Fluor- und/oder Chloratomen und 1 bis 4 Kohlenstoffatomen in jedem Halogenalkylteil, N-Alkyl-N-alkylsulfonyl-amino mit 1 bis 4 Kohlenstoffatomen im Alkylteil und 1 bis 4 Kohlenstoffatomen im Alkylsulfonylteil, N-Halogenalkyl-N-halogenalkylsulfonyl-amino mit 1 bis 3 Fluor- und/oder Chloratomen und 1 bis 4 Kohlenstoffatomen im Halogenalkylteil und mit 1 bis 3 Fluor- und/oder Chloratomen und 1 bis 4 Kohlenstoffatomen im Halogenalkylsulfonylteil, N-Alkylcarbonyl-N-alkylsulfonyl-amino mit 1 bis 4 Kohlenstoffatomen im Alkylteil der Alkylcarbonylgruppe und 1 bis 4 Kohlenstoffatomen im Alkylsulfonylteil, N-Halogenalkylcarbonyl-N-halogenalkylsulfonyl-amino mit 1 bis 3 Fluor- und/oder Chloratomen und 1 bis 4 Kohlenstoffatomen im Halogenalkylteil der Halogenalkylcarbonylgruppe und mit 1 bis 3 Fluor- und/oder Chloratomen und 1 bis 4 Kohlenstoffatomen im Halogenalkylsulfonylteil oder für gegebenenfalls einfach bis dreifach, gleichartig oder verschieden durch Fluor, Chlor, Brom, Methyl, Ethyl, n-Propyl, i-Propyl, n-Butyl, i-Butyl, s-Butyl, t-Butyl, Dimenthylamino, Diethylamino, Methoxy, Ethoxy, n-Propoxy und/oder i-Propoxy substituiertes N-Alkylsulfonyl-N-phenylcarbonyl-amino mit 1 bis 4 Kohlenstoffatomen im Alkylsulfonylteil steht, und
- n: für die Zahlen 0, 1 oder 2 steht, wobei R⁷ für gleiche oder verschiedene Reste steht, wenn n für 2 steht,
oder
- Het: für den Rest der Formel 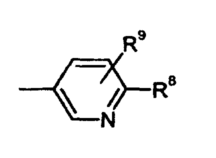 steht, worin
- R⁸: für Cyano steht und
- R⁹: für Alkylsulfonylamino mit 1 bis 4 Kohlenstoffatomen, Halogenalkylsulfonylamino mit 1 bis 3 Fluor- und/oder Chloratomen und 1 bis 4 Kohlenstoffatomen, N,N-Bis-alkylsulfonylamino mit 1 bis 4 Kohlenstoffatomen in jedem Alkylteil, N,N-Bis-halogenalkylsulfonyl-amino mit 1 bis 3 Fluor- und/oder Chloratomen und 1 bis 4 Kohlenstoffatomen in jedem Halogenalkylteil, N-Alkyl-N-alkylsulfonyl-amino mit 1 bis 4 Kohlenstoffatomen im Alkylteil und 1 bis 4 Kohlenstoffatomen im Alkylsulfonylteil, N-Halogenalkyl-N-halogenalkylsulfonyl-amino mit 1 bis 3 Fluor- und/oder Chloratomen und 1 bis 4 Kohlenstoffatomen im Halogenalkylteil und mit 1 bis 3 Fluor- und/oder Chloratomen und 1 bis 4 Kohlenstoffatomen im Halogenalkylsulfonylteil, N-Alkylcarbonyl-N-alkylsulfonyl-amino mit 1 bis 4 Kohlenstoffatomen im Alkylteil der Alkylcarbonylgruppe und 1 bis 4 Kohlenstoffatomen im Alkylsulfonylteil, N-Halogenalkylcarbonyl-N-halogenalkylsulfonyl-amino mit 1 bis 3 Fluor- und/oder Chloratomen und 1 bis 4 Kohlenstoffatomen im Halogenalkylteil der Halogenalkylcarbonylgruppe und mit 1 bis 3 Fluor- und/oder Chloratomen und 1 bis 4 Kohlenstoffatomen im Halogenalkylsulfonylteil oder für gegebenenfalls einfach bis dreifach, gleichartig oder verschieden durch Fluor, Chlor, Brom, Methyl, Ethyl, n-Propyl, i-Propyl, n-Butyl, i-Butyl, s-Butyl, t-Butyl, Dimenthylamino, Diethylamino, Methoxy, Ethoxy, n-Propoxy und/oder i-Propoxy substituiertes N-Alkylsulfonyl-N-phenylcarbonyl-amino mit 1 bis 4 Kohlenstoffatomen im Alkylsulfonylteil steht.

Besonders bevorzugt sind Heterocyclyluracile der Formel (I), in denen
- R¹: für Wasserstoff, Methyl, Ethyl oder Difluormethyl steht,
- R²: für Carboxy, Methoxycarbonyl, Cyano, Carbamoyl, Thiocarbonyl oder für einfach bis dreifach, gleichartig oder verschieden durch Fluor und/oder Chlor substituiertes Methyl oder Ethyl steht,
- R³: für Wasserstoff, Fluor, Chlor, Brom oder Methyl steht und
- Het: für den Rest der Formel 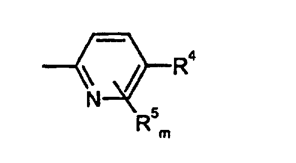 steht, worin
- R⁴: für Cyano oder Thiocarbamoyl steht,
- R⁵: für Alkoxy mit 1 bis 4 Kohlenstoffatomen, Halogenalkoxy mit 1 bis 3 Fluor- und/oder Chloratomen und 1 oder 2 Kohlenstoffatomen, Alkoxyalkoxy mit 1 oder 2 Kohlenstoffatomen in jedem Alkoxyteil, Alkylthio mit 1 oder 2 Kohlenstoffatomen, Halogenalkylthio mit 1 bis 3 Fluor- und/oder Chloratomen und 1 bis 4 Kohlenstoffatomen, Alkylsulfonylamino mit 1 bis 4 Kohlenstoffatomen, Halogenalkylsulfonylamino mit 1 bis 3 Fluor- und/oder Chloratomen und 1 bis 4 Kohlenstoffatomen, N,N-Bis-alkylsulfonylamino mit 1 bis 4 Kohlenstoffatomen in jedem Alkylteil, N,N-Bis-halogenalkylsulfonyl-amino mit 1 bis 3 Fluor- und/oder Chloratomen und 1 bis 4 Kohlenstoffatomen in jedem Halogenalkylteil, N-Alkyl-N-alkylsulfonyl-amino mit 1 bis 4 Kohlenstoffatomen im Alkylteil und 1 bis 4 Kohlenstoffatomen im Alkylsulfonylteil, N-Halogenalkyl-N-halogenalkylsulfonyl-amino mit 1 bis 3 Fluor- und/oder Chloratomen und 1 bis 4 Kohlenstoffatomen im Halogenalkylteil und mit 1 bis 3 Fluor- und/oder Chloratomen und 1 bis 4 Kohlenstoffatomen im Halogenalkylsulfonylteil, N-Alkylcarbonyl-N-alkylsulfonyl-amino mit 1 bis 4 Kohlenstoffatomen im Alkylteil der Alkylcarbonylgruppe und 1 bis 4 Kohlenstoffatomen im Alkylsulfonylteil, N-Halogenalkylcarbonyl-N-halogenalkylsulfonyl-amino mit 1 bis 3 Fluor- und/oder Chloratomen und 1 bis 4 Kohlenstoffatomen im Halogenalkylteil der Halogenaklylcarbonylgruppe und mit 1 bis 3 Fluor- und/oder Chloratomen und 1 bis 4 Kohlenstoffatomen im Halogenalkylsulfonylteil oder für gegebenenfalls einfach bis dreifach, gleichartig oder verschieden durch Fluor, Chlor, Brom, Methyl, Ethyl, Dimethylamino, Diethylamino, Methoxy und/oder Ethoxy substituiertes N-Alkylsulfonyl-N-phenylcarbonyl-amino mit 1 bis 4 Kohlenstoffatomen im Alkylsulfonylteil steht,
und
- m: für die Zahlen 0, 1 oder 2 steht, wobei R⁵ für gleiche oder verschiedene Reste steht, wenn m für 2 steht,
oder
- Het: für den Rest der Formel 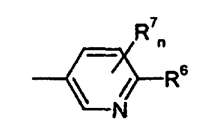 steht, worin
- R⁶: für Carbamoyl oder Thiocarbamoyl steht,
- R⁷: für Alkoxy mit 1 bis 4 Kohlenstoffatomen, Halogenalkoxy mit 1 bis 3 Fluor- und/oder Chloratomen und 1 oder 2 Kohlenstoffatomen, Alkoxyalkoxy mit 1 oder 2 Kohlenstoffatomen in jedem Alkoxyteil, Alkylthio mit 1 oder 2 Kohlenstoffatomen, Halogenalkylthio mit 1 bis 3 Fluor- und/oder Chloratomen und 1 bis 4 Kohlenstoffatomen, Alkylsulfonylamino mit 1 bis 4 Kohlenstoffatomen, Halogenalkylsulfonylamino mit 1 bis 3 Fluor- und/oder Chloratomen und 1 bis 4 Kohlenstoffatomen, N,N-Bis-alkylsulfonylamino mit 1 bis 4 Kohlenstoffatomen in jedem Alkylteil, N,N-Bis-halogenalkylsulfonyl-amino mit 1 bis 3 Fluor- und/oder Chloratomen und 1 bis 4 Kohlenstoffatomen in jedem Halogenalkylteil, N-Alkyl-N-alkylsulfonyl-amino mit 1 bis 4 Kohlenstoffatomen im Alkylteil und 1 bis 4 Kohlenstoffatomen im Alkylsulfonylteil, N-Halogenalkyl-N-halogenalkylsulfonyl-amino mit 1 bis 3 Fluor- und/oder Chloratomen und 1 bis 4 Kohlenstoffatomen im Halogenalkylteil und mit 1 bis 3 Fluor- und/oder Chloratomen und 1 bis 4 Kohlenstoffatomen im Halogenalkylsulfonylteil, N-Alkylcarbonyl-N-alkylsulfonyl-amino mit 1 bis 4 Kohlenstoffatomen im Alkylteil der Alkylcarbonylgruppe und 1 bis 4 Kohlenstoffatomen im Alkylsulfonylteil, N-Halogenalkylcarbonyl-N-halogenalkylsulfonyl-amino mit 1 bis 3 Fluor- und/oder Chloratomen und 1 bis 4 Kohlenstoffatomen im Halogenalkylteil der Halogenalkylcarbonylgruppe und mit 1 bis 3 Fluor- und/oder Chloratomen und 1 bis 4 Kohlenstoffatomen im Halogenalkylsulfonylteil oder für gegebenenfalls einfach bis dreifach, gleichartig oder verschieden durch Fluor, Chlor, Brom, Methyl, Ethyl, Dimethylamino, Diethylamino, Methoxy und/oder Ethoxy substituiertes N-Alkylsulfonyl-N-phenylcarbonyl-amino mit 1 bis 4 Kohlenstoffatomen im Alkylsulfonylteil steht,
und
- n: für die Zahlen 0, 1 oder 2 steht, wobei R⁷ für gleiche oder verschiedene Reste steht, wenn n für 2 steht,
oder
- Het: für den Rest der Formel 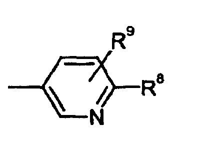 steht, worin
- R⁸: für Cyano steht und
- R⁹: für Alkylsulfonylamino mit 1 bis 4 Kohlenstoffatomen, Halogenalkylsulfonylamino mit 1 bis 3 Fluor- und/oder Chloratomen und 1 bis 4 Kohlenstoffatomen, N,N-Bis-alkylsulfonylamino mit 1 bis 4 Kohlenstoffatomen in jedem Alkylteil, N,N-Bis-halogenalkylsulfonyl-amino mit 1 bis 3 Fluor- und/oder Chloratomen und 1 bis 4 Kohlenstoffatomen in jedem Halogenalkylteil, N-Alkyl-N-alkylsulfonyl-amino mit 1 bis 4 Kohlenstoffatomen im Alkylteil und 1 bis 4 Kohlenstoffatomen im Alkylsulfonylteil, N-Halogenalkyl-N-halogenalkylsulfonyl-amino mit 1 bis 3 Fluor- und/oder Chloratomen und 1 bis 4 Kohlenstoffatomen im Halogenalkylteil und mit 1 bis 3 Fluor- und/oder Chloratomen und 1 bis 4 Kohlenstoffatomen im Halogenalkylsulfonylteil, N-Alkylcarbonyl-N-alkylsulfonyl-amino mit 1 bis 4 Kohlenstoffatomen im Alkylteil der Alkylcarbonylgruppe und 1 bis 4 Kohlenstoffatomen im Alkylsulfonylteil, N-Halogenalkylcarbonyl-N-halogenalkylsulfonyl-amino mit 1 bis 3 Fluor- und/oder Chloratomen und 1 bis 4 Kohlenstoffatomen im Halogenalkylteil der Halogenaklylcarbonylgruppr und mit 1 bis 3 Fluor- und/oder Chloratomen und 1 bis 4 Kohlenstoffatomen im Halogenalkylsulfonylteil oder für gegebenenfalls einfach bis dreifach, gleichartig oder verschieden durch Fluor, Chlor, Brom, Methyl, Ethyl, Dimethylamino, Diethylamino, Methoxy und/oder Ethoxy substituiertes N-Alkylsulfonyl-N-phenylcarbonyl-amino mit 1 bis 4 Kohlenstoffatomen im Alkylsulfonylteil steht.

Ganz besonders bevorzugt sind Heterocyclyluracile der Formel (I), in denen
- R¹: für Wasserstoff, Methyl, Ethyl oder Difluormethyl steht,
- R²: für Carboxy, Methoxycarbonyl, Cyano, Carbamoyl, Thiocarbamoyl, Methyl oder Trifluormethyl steht,
- R³: für Wasserstoff steht und
- Het: für einen heterocyclischen Rest der folgenden Formeln steht:

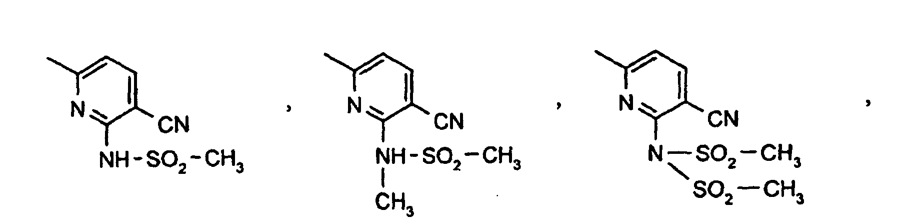

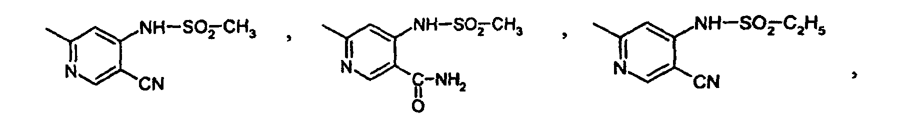

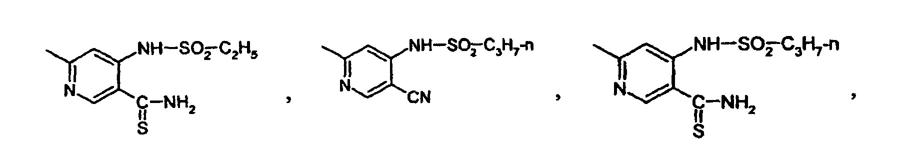

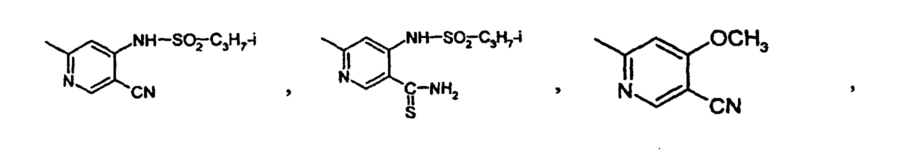

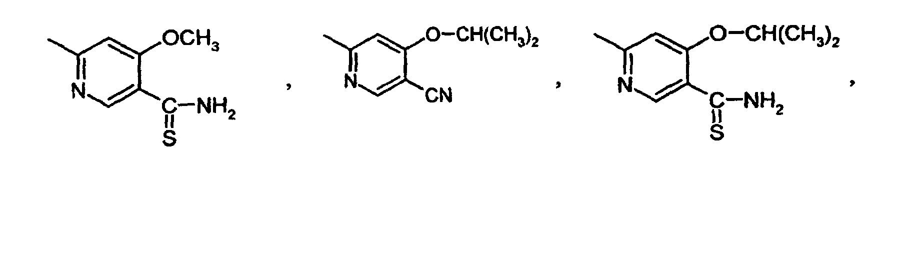

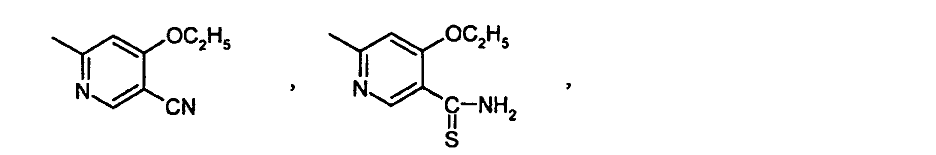

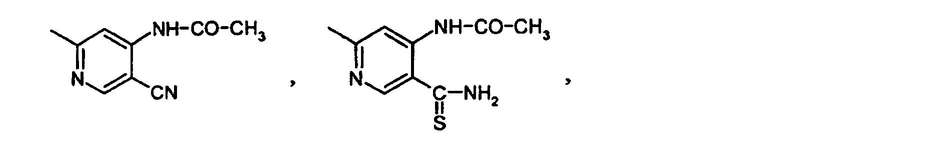

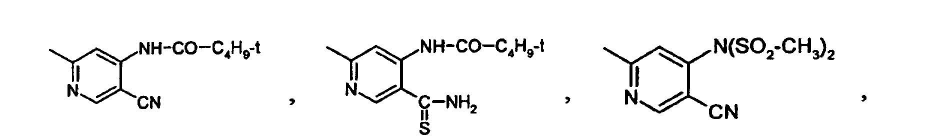

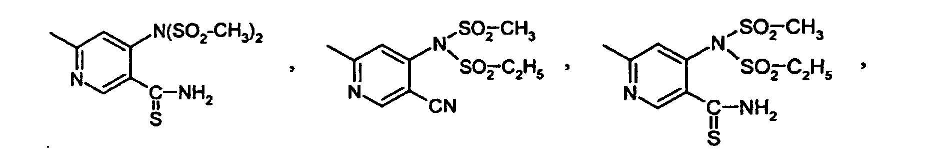

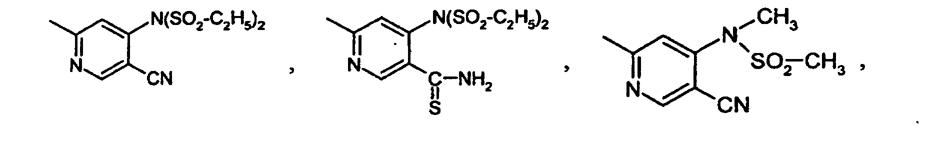

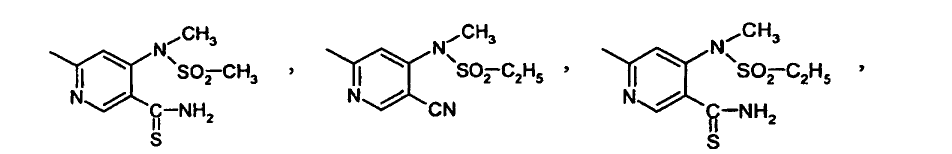

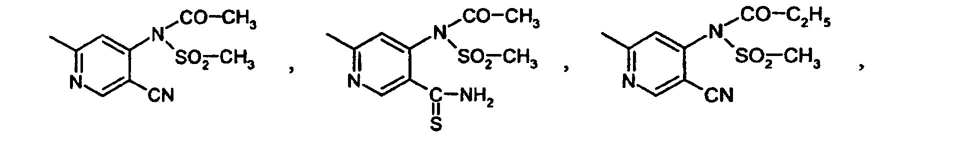

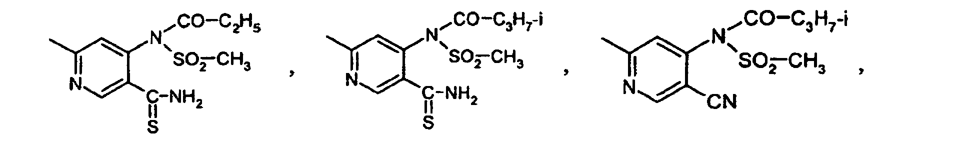

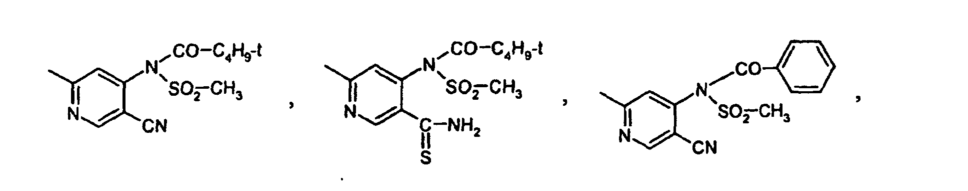

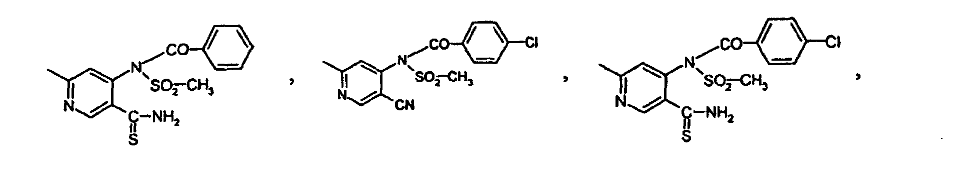

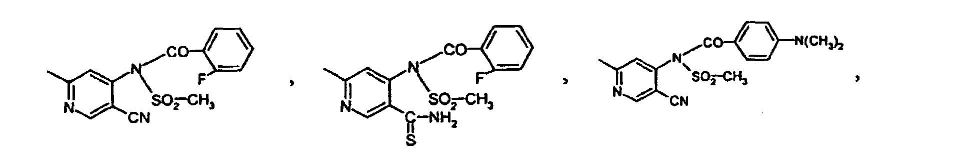

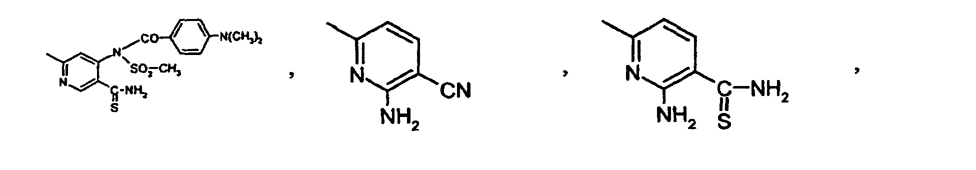

Die oben angegebenen Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen Bereichen beliebig kombiniert werden.

Die erfindungsgemäßen Heterocyclyluracile der Formel (I), in denen R¹ für Wasserstoff steht, können in der "Keto"-Form der Formel 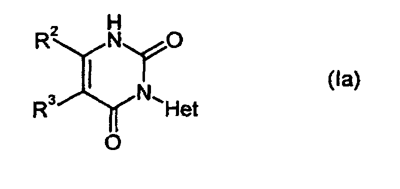 oder in der tautomeren "Enol"-Form der Formel 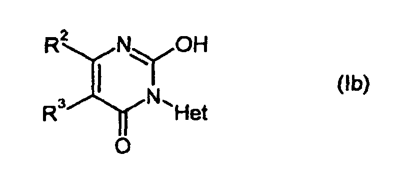 vorliegen. Der Einfachheit halber wird jeweils nur die "Keto"-Form aufgeführt.

Die bei der Durchführung des erfindungsgemäßen Verfahrens (a) als Ausgangsstoffe benötigten Aminoalkensäureester sind durch die Formel (II) allgemein definiert. In der Formel (II) haben R² und R³ vorzugsweise bzw. insbesondere diejenigen Bedeutungen, die bereits oben im Zusammenhang mit der Beschreibung der erfindungsgemäßen Verbindungen der Formel (I) vorzugsweise bzw. besonders bevorzugt für R² und R³ angegeben wurden. R steht vorzugsweise für Alkyl mit 1 bis 4 Kohlenstoffatomen, Phenyl oder Benzyl, besonders bevorzugt für Methyl, Ethyl, Phenyl oder Benzyl. R¹⁰ steht vorzugsweise für Alkyl mit 1 bis 3 Kohlenstoffatomen, besonders bevorzugt für Methyl oder Ethyl.

Die Aminoalkensäureester der Formel (II) sind bekannt oder können nach an sich bekannten Verfahren hergestellt werden (vgl. J. Heterocycl. Chem. 9 (1972), 513-522).

Die bei der Durchführung des erfindungsgemäßen Verfahrens (a. nach Variante α) als Reaktionskomponenten benötigten Heterocyclylisocyanate sind durch die Formel (III) allgemein definiert. In der Formel (III) hat Het vorzugsweise bzw. insbesondere diejenigen Bedeutungen, die bereits oben im Zusammenhang mit der Beschreibung der erfindungsgemäßen Verbindungen der Formel (I) vorzugsweise bzw. besonders bevorzugt für Het angegeben wurden.

Die Heterocyclylisocyanate der Formel (III) sind bekannt oder lassen sich nach prinzipiell bekannten Verfahren herstellen (vgl. EP-A 0 555 770 und EP-A 0 600 836). So lassen sich Heterocyclylisocyanate der Formel (III) herstellen, indem man Heterocyclylamine der Formel

H₂N-Het (VII),

in welcher
- Het: die oben angegebene Bedeutung hat,
mit Phosgen in Gegenwart eines Verdünnungsmittels, wie z.B. Chlorbenzol, bei Temperaturen zwischen -20°C und + 150°C umsetzt.

Die Heterocyclylamine der Formel (VII) sind bekannt oder lassen sich nach prinzipiell bekannten Verfahren herstellen.

Die bei der Durchführung des erfindungsgemäßen Verfahrens (a, nach Variante β) als Reaktionskomponenten benötigten Heterocyclylcarbamate sind durch die Formel (IV) allgemein definiert. In der Formel (IV) hat Het vorzugsweise bzw. insbesondere diejenigen Bedeutungen, die bereits oben im Zusammenhang mit der Beschreibung der erfindungsgemäßen Verbindungen der Formel (I) vorzugsweise bzw. besonders bevorzugt für Het angegeben wurden. R¹¹ steht vorzugsweise für C₁-C₄-Alkyl, Phenyl oder Benzyl, insbesondere für Methyl, Ethyl oder Phenyl.

Die Heterocyclylcarbamate der Formel (IV) sind bekannt oder lassen sich nach prinzipiell bekannten Verfahren herstellen (vgl, EP-A 0 555 770 und EP-A 0 600 836). So erhält man Heterocyclylcarbamate der Formel (IV), wenn man Heterocyclylamine der Formel

H₂N-Het (VII),

in welcher
- Het: die oben angegebene Bedeutung hat,
mit Chlorcarbonylverbindungen der Formel

R¹¹O-CO-Cl (VIII),

in welcher
- R¹¹: die oben angegebene Bedeutung hat,
gegebenenfalls in Gegenwart eines Säureakzeptors, wie z.B. Pyridin, und gegebenenfalls in Gegenwart eines Verdünnungsmittels, wie z.B. Methylenchlorid bei Temperaturen zwischen -20°C und +100°C umsetzt.

Die bei der Durchführung des erfindungsgemäßen Verfahrens (b) als Ausgangsstoffe benötigten Heterocyclyluracile sind durch die Formel (Ia) allgemein definiert. Es handelt sich hierbei um Stoffe, die nach dem erfindungsgemäßen Verfahren (a) herstellbar sind.

Die bei der Durchführung des erfindungsgemäßen Verfahrens (b, Variante α) als Reaktionskomponenten benötigten Halogenverbindungen sind durch die Formel (V) allgemein definiert. In dieser Formel steht R¹² vorzugsweise für gegebenenfalls durch Cyano, Fluor, Chlor, Methoxy oder Ethoxy substituiertes Alkyl mit I bis 3 Kohlenstoffatomen. Hal steht vorzugsweise für Brom oder Iod.

Besonders bevorzugt verwendbar sind Halogenverbindungen der Formel (V), in denen
- R¹²: für Methyl, Ethyl oder Difluormethyl steht und
- Hal: für Brom oder Iod steht.

Die Halogenverbindungen der Formel (V) sind bekannt oder lassen sich nach prinzipiell bekannten Verfahren herstellen.

Die bei der Durchführung des erfindungsgemäßen Verfahrens (b, Variante β) als Reaktionskomponenten benötigten Dialkylsulfate sind durch die Formel (VI) allgemein definiert. In dieser Formel steht R¹³ vorzugsweise für Alkyl mit 1 bis 3 Kohlenstoffatomen, besonders bevorzugt für Methyl oder Ethyl.

Die Dialkylsulfate der Formel (VI) sind bekannt.

Als Säureakzeptoren kommen bei der Durchführung des erfindungsgemäßen Verfahrens (a) sowohl nach Variante (α) als auch nach Variante (β) alle üblichen anorganischen und organischen Basen in Betracht. Vorzugsweise verwendbar sind Alkalimetall- oder Erdalkalimetall- -acetate, -amide, -carbonate, -hydrogencarbonate, -hydride, -hydroxide oder -alkanolate, wie Natrium-, Kalium- oder Calciumacetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, -n- oder -i-propanolat, -n-, -i-, -s- oder -t-butanolat; weiterhin auch basische organische Stickstoffverbindungen, wie Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylaminopyridin, N-Methyl-piperidin, 1,4-Diazabicyclo[2,2,2]-octan (DABCO), 1,5-Diazabicyclo[4,3,0]-non-5-en (DBN), oder 1,8 Diazabicyclo[5,4,0]-undec-7-en (DBU).

Als Verdünnungsmittel kommen bei der Durchführung des erfindungsgemäßen Verfahrens (a) nach Varianten (α) oder (β) alle üblichen inerten, organischen Solventien und auch Wasser in Frage. Vorzugsweise verwendbar sind aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, oder Sulfoxide, wie Dimethylsulfoxid.

Die Reaktionstemperaturen können bei der Durchführung des erfindungsgemäßen Verfahrens (a) innerhalb eines größeren Bereiches variiert werden. Sowohl bei der Durchführung der Variante (α) als auch der Variante (β) arbeitet man im allgemeinen zwischen 0°C und 200°C, vorzugsweise zwischen 10°C und 150°C.

Bei der Durchführung des erfindungsgemäßen Verfahrens (a) nach Varianten (α) und (β) arbeitet man im allgemeinen unter Atmosphärendruck. Es ist jedoch jeweils auch möglich, unter erhöhtem oder vermindertem Druck, z.B. zwischen 0,1 und 10 bar, zu arbeiten.

Bei der Durchführung des erfindungsgemäßen Verfahrens (a) setzt man auf 1 mol an Aminoalkensäureester der Formel (II) im Falle der Variante (α) eine etwa äquimolare Menge an Heterocyclylisocyanat der Formel (III) und im Falle der Variante (β) eine angenähert äquimolare Menge an Heterocyclylcarbamat der Formel (IV) ein. Es ist jedoch auch möglich, jeweils eine der Komponenten in einem größerem Überschuß zu verwenden. Die Umsetzungen werden im allgemeinen in einem geeigneten Verdünnungsmittel und in Gegenwart eines Säurebindemittels durchgeführt. Man verfährt dabei in der Weise, daß man das Reaktionsgemisch so lange wie nötig bei der erforderlichen Temperatur rührt und dann nach üblichen Methoden aufarbeitet.

Als Säurebindemittel kommen bei der Durchführung des erfindungsgemäßen Verfahrens (b) nach den Varianten (α) oder (β) alle üblichen anorganischen und organischen Basen in Betracht. Vorzugsweise verwendbar sind diejenigen Säureakzeptoren, die bereits im Zusammenhang mit der Beschreibung des erfindungsgemäßen Verfahrens (a) vorzugsweise genannt wurden.

Als Verdünnungsmittel kommen bei der Durchführung des erfindungsgemäßen Verfahrens (b) nach den Varianten (α) oder (β) alle für derartige Umsetzungen üblichen inerten, organischen Solventien in Frage. Vorzugsweise verwendbar sind Nitrile, wie Acetonitril und Butyronitril, ferner Ketone, wie Aceton, und außerdem Amide, wie Dimethylformamid und N-Methyl-pyrrolidon.

Die Reaktionstemperaturen können auch bei der Durchführung des erfindungsgemäßen Verfahrens (b) innerhalb eines größeren Bereiches variiert werden. Man arbeitet bei der Durchführung
- der Variante (α) im allgemeinen bei Temperaturen zwischen 0°C und 150°C, vorzugsweise zwischen 10°C und 120°C,
- der Variante (β) im allgemeinen bei Temperaturen zwischen 10°C und 100°C, vorzugsweise zwischen 15°C und 80°C.

Auch bei der Durchführung des erfindungsgemäßen Verfahrens (b) nach den Varianten (α) und (β) arbeitet man im allgemeinen unter Atmosphärendruck. Es ist jedoch auch möglich, jeweils unter erhöhtem oder, sofern keine flüchtigen Komponenten an der Umsetzung beteiligt sind, unter vermindertem Druck zu arbeiten.

Bei der Durchführung des erfindungsgemäßen Verfahrens (b) nach den Varianten (α) und (β) setzt man auf 1 mol an Heterocyclyluracil der Formel (Ia) im allgemeinen eine angenähert äquimolare Menge an Halogenverbindung der Formel (V) bzw. an Dialkylsulfat der Formel (VI) ein. Es ist jedoch auch möglich, jeweils eine der Komponenten in einem größeren Überschuß zu verwenden. Die Aufarbeitung erfolgt jeweils nach üblichen Methoden.

Die erfindungsgemäßen Wirkstoffe weisen eine sehr gute herbizide Wirksamkeit auf und können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattuneen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium. Veronica, Abutiton, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum. Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen Kulturen sowohl im Vorauflauf- als auch im Nachauflauf-Verfahren. Sie zeigen auch eine gute Verträglichkeit gegenüber wichtigen Kulturpflanzen, wie Mais und Weizen.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind. Dabei kann in manchen Fällen auch Synergismus auftreten.

Für die Mischungen kommen beispielsweise folgende Herbizide infrage:

Acetochlor, Acifluorfen(-sodium), Aclonifen, Alachlor, Alloxydim(-sodium), Ametryne, Amidochlor, Amidosulfuron, Asulam, Atrazine, Azimsulfuron, Benazolin, Benfuresate, Bensulfuron(-methyl), Bentazon, Benzofenap, Benzoylprop(-ethyl), Bialaphos, Bifenox, Bromobutide, Bromofenoxim, Bromoxynil, Butachlor, Butylate, Cafenstrole, Carbetamide, Chlomethoxyfen, Chloramben, Chloridazon, Chlorimuron(-ethyl), Chlornitrofen, Chlorsulfuron, Chlortoluron, Cinmethylin, Cinosulfuron, Clethodim, Clodinafop(-propargyl), Clomazone, Clopyralid, Clopyrasulfuron, Cloransulam(-methyl), Cumyluron, Cyanazine, Cycloate, Cyclosulfamuron, Cycloxydim, Cyhalofop(-butyl), 2,4-D, 2,4-DB, 2,4-DP, Desmedipham, Diallate, Dicamba, Diclofop(-methyl), Difenzoquat, Diflufenican, Dimefuron, Dimepiperate, Dimethachlor, Dimethametryn, Dimethenamid, Dinitramine, Diphenamid, Diquat, Dithiopyr, Diuron, Dymron, EPTC, Esprocarb, Ethalfluralin, Ethametsulfuron(-methyl), Ethofurnesate, Ethoxyfen, Etobenzanid, Fenoxaprop(-ethyl), Flamprop(-isopropyl), Flamprop(-isopropyl-L), Flamprop(-methyl), Flazasulfuron, Fluazifop(-butyl), Flumetsulam, Flumiclorac(-pentyl), Flumioxazin, Flumipropyn, Fluometuron, Fluorochloridone, Fluoroglycofen(-ethyl), Flupoxam, Flupropacil, Flurenol, Fluridone, Fluroxypyr, Flurprimidol, Flurtamone, Fomesafen, Glufosinate(-ammonium), Glyphosate(-isopropylammonium), Halosafen, Haloxyfop(-ethoxyethyl), Hexazinone, Imazamethabenz(-methyl), Imazamethapyr, Imazamox, Imazapyr, Imazaquin, Imazethapyr, Imazosulfuron, loxynil, Isopropalin, Isoproturon, Isoxaben, Isoxaflutole, Isoxapyrifop, Lactofen, Lenacil, Linuron, MCPA, MCPP, Mefenacet, Metamitron, Metazachlor, Methabenzthiazuron, Metobenzuron, Metobromuron, Metolachlor, Metosulam, Metoxuron, Metribuzin, Metsulfuron(-methyl), Molinate, Monolinuron, Naproanilide, Napropamide, Neburon, Nicosulfuron, Norflurazon Orbencarb, Oryzalin, Oxadiazon, Oxyfluorfen, Paraquat, Pendimethalin. Phenmedipham, Piperophos, Pretilachlor, Primisulfuron(-methyl), Prometryn, Propachlor, Propanil, Propaquizafop, Propyzamide, Prosulfocarb, Prosulfuron, Pyrazolate, Pyrazosulfuron(-ethyl), Pyrazoxyfen, Pyributicarb, Pyridate, Pyrithiobac(-sodium), Quinchlorac, Quinmerac, Quizalofop(-ethyl), Quizalofop(-p-tefuryl), Rimsulfuron, Sethoxydim, Simazine, Simetryn, Sulcotrione, Sulfentrazone, Sulfometuron(-methyl), Sulfosate, Tebutam, Tebuthiuron, Terbuthylazine, Terbutryn, Thenylchlor, Thiafluamide, Thiazopyr, Thidiazimin, Thifensulfuron(-methyl), Thiobencarb, Tiocarbazil, Tralkoxydim, Triallate, Triasulfuron, Tribenuron(-methyl), Triclopyr, Tridiphane, Trifluralin und Triflusulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise. z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele

Nach den zuvor angegebenen Methoden werden die in der nachstehenden Tabelle 1 aufgeführten Verbindungen der Formel (I) hergestellt.

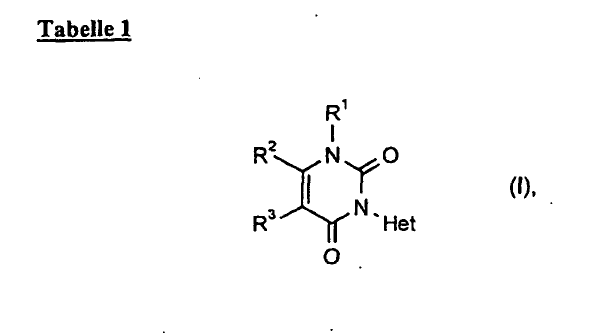

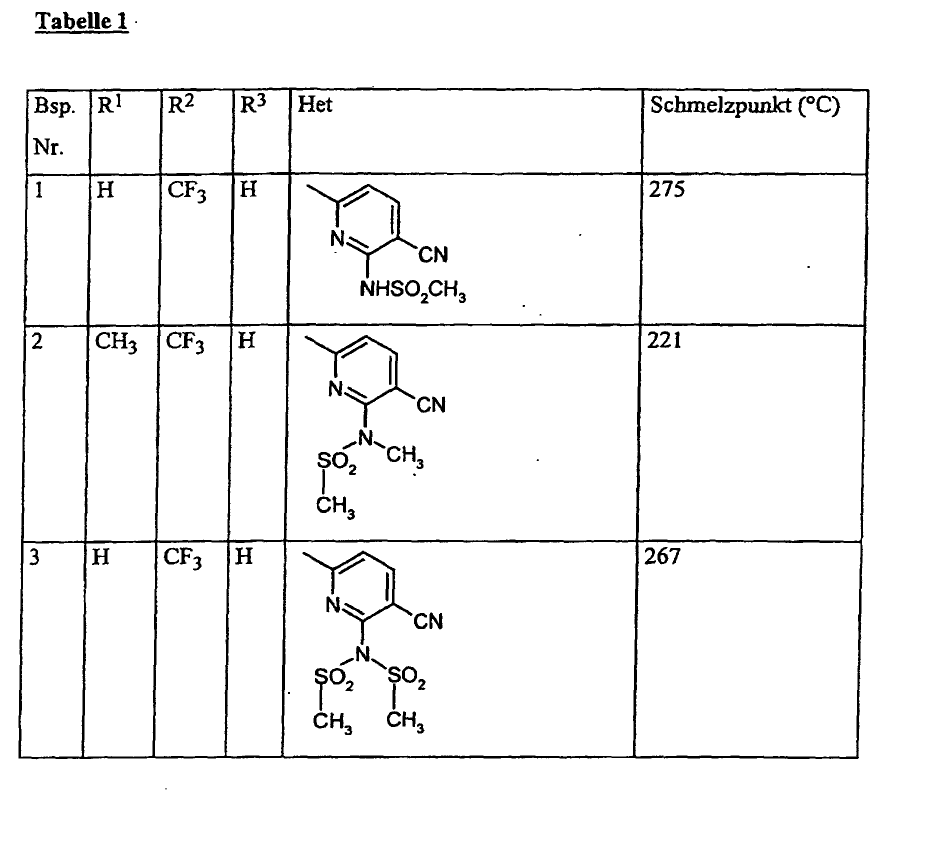

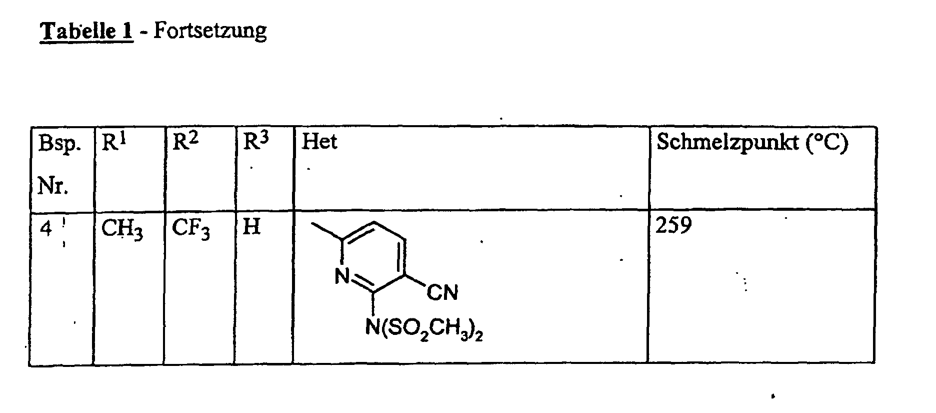

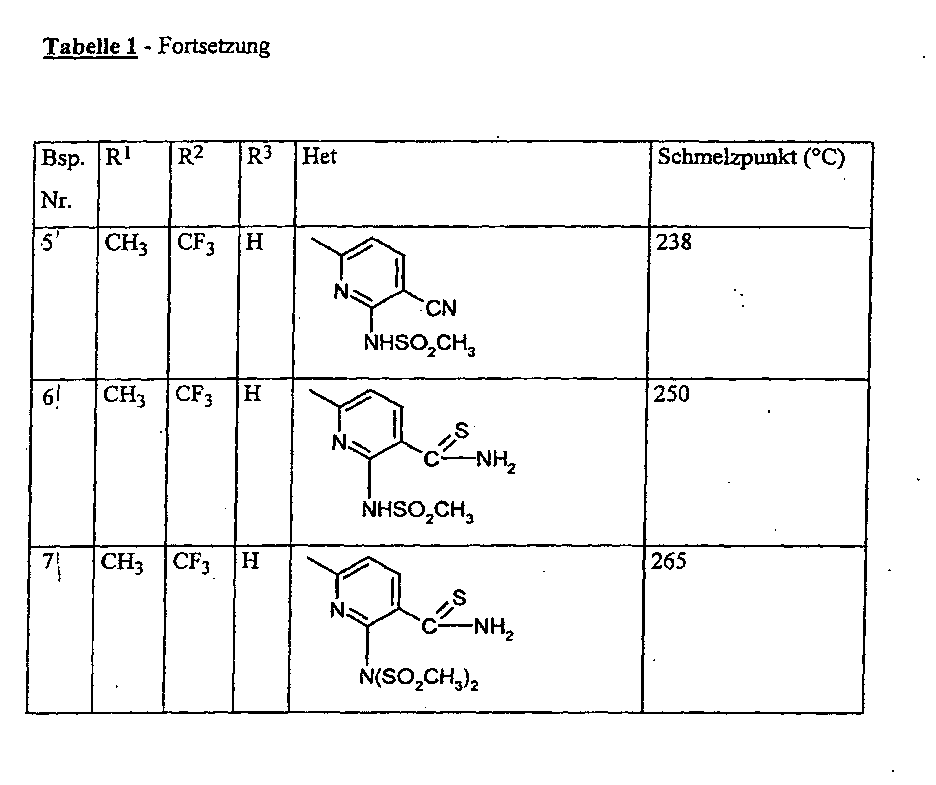

### Verwendungsbeispiele:

### Beispiel A

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach 24 Stunden wird der Boden mit der Wirkstoffzubereitung so gespritzt, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

In diesem Test zeigt die Verbindung gemäß Herstellungsbeispiel 2 bei Aufwandmengen von 125 bis 250 g/ha bei teilweise guter Verträglichkeit gegenüber Kulturpflanzen, wie Mais und Soja, eine starke Wirkung gegen Unkräuter.

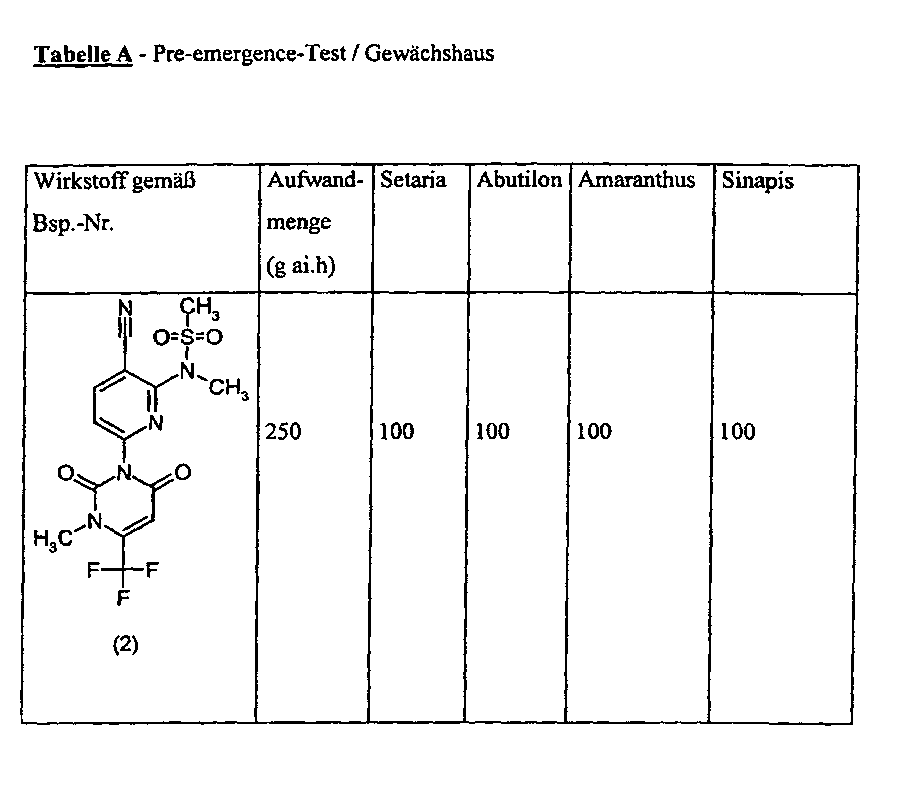

### Beispiel B

### Post-emergence-Test

| | |
|---|---|
| Lösungsmittel: | 5 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5-15 cm haben so, daß die jeweils gewünschten Wirkstoffmengen pro Flächeneinheit ausgebracht werden. Die Konzentration der Spritzbrühe wird so gewählt, daß in 1000 1 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

In diesem Test zeigt die Verbindung gemäß Herstellungsbeispiel 2 bei Aufwandmengen von 60 bis 250 g/ha bei teilweise guter Verträglichkeit gegenüber Kulturpflanzen, wie Weizen, eine starke Wirkung gegen Unkräuter.

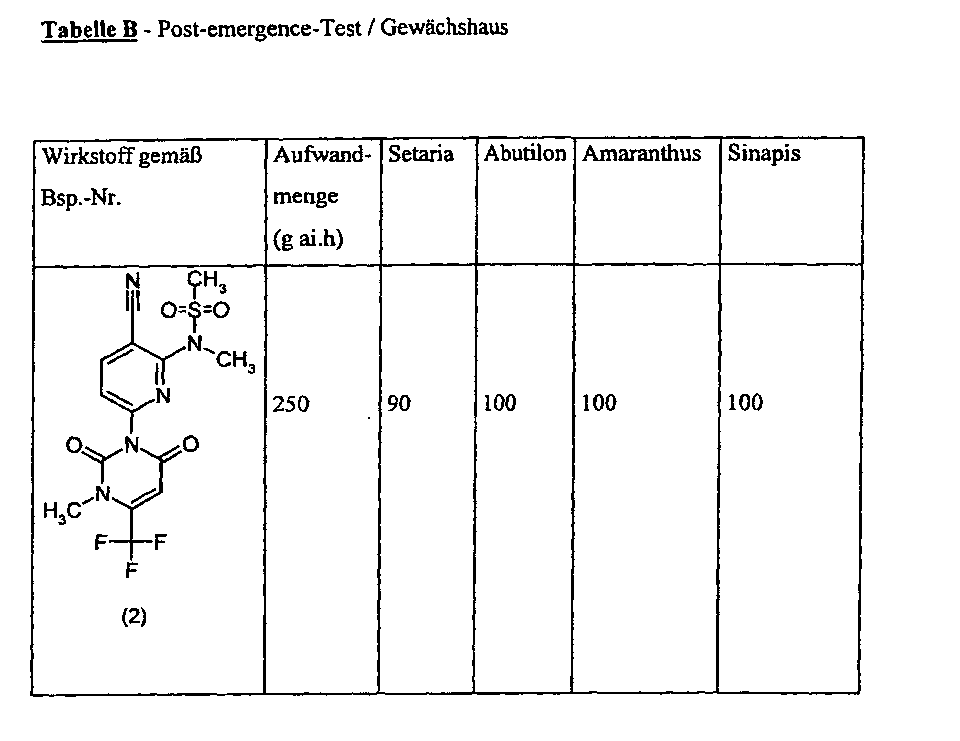

## Patentansprüche

1. Heterocyclyluracile der Formel 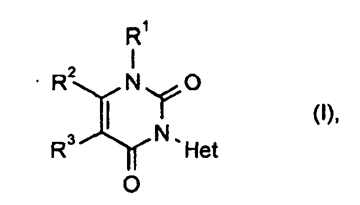 in welcher
R¹ für Wasserstoff oder für gegebenenfalls durch Cyano, Halogen oder C₁-C₄-Alkoxy substituiertes C₁-C₄-Alkyl steht,
R² für Formyl, Hydroximinomethyl, Cyano, Carboxy, Alkoxycarbonyl, Carbamoyl, Thiocarbamoyl oder für gegebenenfalls durch Halogen substituiertes C₁-C₄-Alkyl steht,
R³ für Wasserstoff, Cyano, Halogen oder für gegebenenfalls durch Halogen substituiertes C₁-C₄-Alkyl steht und
Het für den Rest der Formel 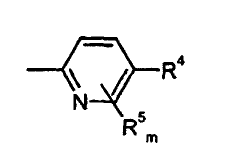 steht, worin
R⁴ für Cyano oder Thiocarbamoyl steht,
R⁵ für Alkoxy mit 1 bis 6 Kohlenstoffatomen, Halogenalkoxy mit 1 bis 6 Kohlenstoffatomen und 1 bis 5 Halogenatomen, Alkoxyalkoxy mit 1 bis 6 Kohlenstoffatomen in jedem Alkoxyteil, Alkylthio mit 1 bis 6 Kohlenstoffatomen, Halogenalkylthio mit 1 bis 6 Kohlenstoffatomen und 1 bis 5 Halogenatomen, Alkylsulfonylamino mit 1 bis 6 Kohlenstoffatomen im Alkylteil, Halogenalkylsulfonylamino mit 1 bis 5 Halogenatomen und 1 bis 6 Kohlenstoffatomen im Halogenalkylteil, N,N-Bis-alkylsulfonylamino mit 1 bis 6 Kohlenstoffatomen in jedem Alkylteil, N,N-Bis-Halogenalkylsulfonyl-amino mit 1 bis 5 Halogenatomen und 1 bis 6 Kohlenstoffatomen in jedem Halogenalkylteil, N-Alkyl-N-alkylsulfonyl-amino mit 1 bis 6 Kohlenstoffatomen im Alkylteil und 1 bis 6 Kohlenstoffatomen im Alkylsulfonylteil, N-Halogenalkyl-N-halogenalkylsulfonyl-amino mit 1 bis 5 Halogenatomen und 1 bis 6 Kohlenstoffatomen im Halogenalkylteil und 1 bis 5 Halogenatomen und 1 bis 6 Kohlenstoffatomen im Halogenalkylsulfonylteil, N-Alkylcarbonyl-N-alkylsulfonyl-amino mit 1 bis 6 Kohlenstoffatomen im Alkylteil der Alkylcarbonylgruppe und 1 bis 6 Kohlenstoffatomen im Alkylsulfonylteil, N-Halogenalkylcarbonyl-N-halogenalkylsulfonyl-amino mit 1 bis 5 Halogenatomen und 1 bis 6 Kohlenstoffatomen im Halogenalkylteil und 1 bis 5 Halogenatomen und 1 bis 6 Kohlenstoffatomen im Halogenalkylsulfonylteil oder für gegebenenfalls einfach bis dreifach, gleichartig oder verschieden durch Halogen, Alkyl mit 1 bis 4 Kohlenstoffatomen, Dialkylamino mit 1 bis 4 Kohlenstoffatomen in jeder Alkylgruppe und/oder Alkoxy mit 1 bis 4 Kohlenstoffatomen substituiertes N-Alkylsulfonyl-N-phenylcarbonyl-amino mit 1 bis 6 Kohlenstoffatomen im Alkylsulfonylteil steht, und
m für ganze Zahlen von 0 bis 3 steht,
oder
Het für den Rest der Formel 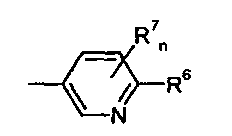 steht, worin
R⁶ für Carbamoyl oder Thiocarbamoyl steht,
R⁷ für Alkoxy mit 1 bis 6 Kohlenstoffatomen, Halogenalkoxy mit 1 bis 6 Kohlenstoffatomen und 1 bis 5 Halogenatomen, Alkoxyalkoxy mit 1 bis 6 Kohlenstoffatomen in jedem Alkoxyteil, Alkylthio mit 1 bis 6 Kohlenstoffatomen, Halogenalkylthio mit 1 bis 6 Kohlenstoffatomen und 1 bis 5 Halogenatomen, Alkylsulfonylamino mit 1 bis 6 Kohlenstoffatomen im Alkylteil, Halogenalkylsulfonylamino mit 1 bis 5 Halogenatomen und 1 bis 6 Kohlenstoffatomen im Halogenalkylteil, N,N-Bis-alkylsulfonylamino mit 1 bis 6 Kohlenstoffatomen in jedem Alkylteil, N,N-Bis-Halogenalkylsulfonyl-amino mit 1 bis 5 Halogenatomen und 1 bis 6 Kohlenstoffatomen in jedem Halogenalkylteil, N-Alkyl-N-alkylsulfonyl-amino mit 1 bis 6 Kohlenstoffatomen im Alkylteil und 1 bis 6 Kohlenstoffatomen im Alkylsulfonylteil, N-Halogenalkyl-N-halogenalkylsulfonyl-amino mit 1 bis 5 Halogenatomen und 1 bis 6 Kohlenstoffatomen im Halogenalkylteil und 1 bis 5 Halogenatomen und 1 bis 6 Kohlenstoffatomen im Halogenalkylsulfonylteil, NAlkylcarbonyl-N-alkylsulfonyl-amino mit 1 bis 6 Kohlenstoffatomen im Alkylteil der Alkylcarbonylgruppe und 1 bis 6 Kohlenstoffatomen im Alkylsulfonylteil, N-Halogenalkylcarbonyl-N-halogenalkylsulfonyl-amino mit 1 bis 5 Halogenatomen und 1 bis 6 Kohlenstoffatomen im Halogenalkylteil und 1 bis 5 Halogenatomen und 1 bis 6 Kohlenstoffatomen im Halogenalkylsulfonylteil oder für gegebenenfalls einfach bis dreifach, gleichartig oder verschieden durch Halogen, Alkyl mit 1 bis 4 Kohlenstoffatomen, Dialkylamine mit 1 bis 4 Kohlenstoffatomen in jeder Alkylgruppe und/oder Alkoxy mit 1 bis 4 Kohlenstoffatomen substituiertes N-Alkylsulfonyl-N-phenylcarbonyl-amino mit 1 bis 6 Kohlenstoffatomen im Alkylsulfonylteil steht, und
n für ganze Zahlen von 0 bis 3 steht,
oder
Het für den Rest der Formel 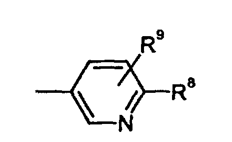 steht,worin
R⁸ für Cyano steht,
R⁹ für Alkylsulfonylamino mit 1 bis 6 Kohlenstoffatomen im Alkylteil, Halogenalkylsulfonylamino mit 1 bis 5 Halogenatomen und 1 bis 6 Kohlenstoffatomen im Halogenalkylteil, N,N-Bis-alkylsulfonylamino mit 1 bis 6 Kohlenstoffatomen in jedem Alkylteil, N,N-Bis-Halogenallcylsulfonyl-amino mit 1 bis 5 Halogenatomen und 1 bis 6 Kohlenstoffatomen in jedem Halogenalkylteil, N-Alkyl-N-alkylsulfonyl-amino mit 1 bis 6 Kohlenstoffatomen im Alkylteil und 1 bis 6 Kohlenstoffatomen im Alkylsulfonylteil, N-Halogenalkyl-N-halogenalkylsulfonyl-amino mit 1 bis 5 Halogenatomen und 1 bis 6 Kohlenstoffatomen im Halogenalkylteil und 1 bis 5 Halogenatomen und 1 bis 6 Kohlenstoffatomen im Halogenalkylsulfonylteil, N-Alkylcarbonyl-N-alkylsulfonyl-amino mit 1 bis 6 Kohlenstoffatomen im Alkylteil der Alkylcarbonylgruppe und 1 bis 6 Kohlenstoffatomen im Alkylsulfonylteil, N-Halogenalkylcarbonyl-N-halogenalkylsulfonyl-amino mit 1 bis 5 Halogenatomen und 1 bis 6 Kohlenstoffatomen im Halogenalkylteil und 1 bis 5 Halogenatomen und 1 bis 6 Kohlenstoffatomen im Halogenalkylsulfonylteil oder für gegebenenfalls einfach bis dreifach, gleichartig oder verschieden durch Halogen, Alkyl mit 1 bis 4 Kohlenstoffatomen, Dialkylamino mit 1 bis 4 Kohlenstoffatomen in jeder Alkylgruppe und/oder Alkoxy mit 1 bis 4 Kohlenstoffatomen substituiertes N-Alkylsulfonyl-N-phenylcarbonyl-amino mit 1 bis 6 Kohlenstoffatomen im Alkylsulfonylteil steht.

2. Verfahren zur Herstellung von Heterocyclyluracilen der Formel (I) gemäß Anspruch 1, **dadurch gekennzeichnet, dass** man
a) Aminoalkensäureester der Formel 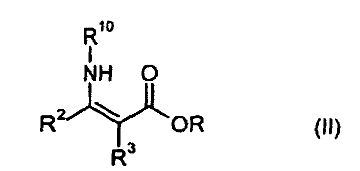 in welcher
R² und R³ die in Anspruch 1 angegebenen Bedeutungen haben,
R¹⁰ für Wasserstoff oder Alkyl mit 1 bis 4 Kohlenstoffatomen steht und
R für Alkyl mit 1 bis 4 Kohlenstoffatomen, Phenyl oder Benzyl steht
entweder
α) mit Heterocyclylisocyanaten der Formel
O=C=N-Het (III),
in welcher
Het die in Anspruch 1 angegebene Bedeutung hat,
oder
β) mit Heterocyclylcarbamaten der Formel 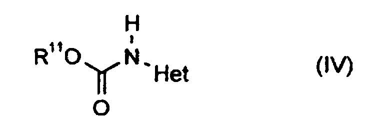
in welcher
Het die in Anspruch 1 angegebene Bedeutung hat und
R¹¹ für Alkyl mit 1 bis 4 Kohlenstoffatomen, Phenyl oder Benzyl steht,
jeweils gegebenenfalls in Gegenwart eines Säurebindemittels und gegebenenfalls in Gegenwart eines Verdünnungsmittels umsetzt,
oder
b) Heterocyclyluracile der Formel 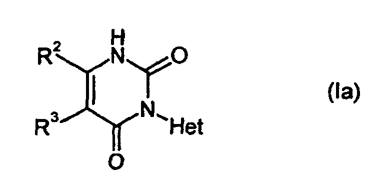
in welcher
R², R³ und Het die in Anspruch 1 angegebenen Bedeutungen haben,
entweder
α) mit Halogenverbindungen der Formel
R¹²-Hal (V)
in welcher
R¹² für gegebenenfalls durch Cyano, Halogen oder Alkoxy mit 1 bis 4 Kohlenstoffatomen substituiertes Alkyl mit 1 bis 4 Kohlenstoffatomen steht und
Hal für Chlor, Brom oder Iod steht,
oder
β) mit Dialkylsulfaten der Formel 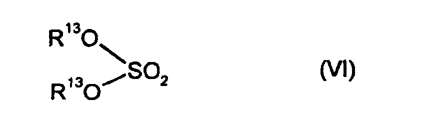
in welcher
R¹³ für Alkyl mit 1 bis 4 Kohlenstoffatomen steht,
jeweils gegebenenfalls in Gegenwart eines Säurebindemittels und gegebenenfalls in Gegenwart eines Verdünnungsmittels umsetzt.

3. Herbizide Mittel, **gekennzeichnet durch** einen Gehalt an mindestens einem Heterocyclyluracil der Formel (I) gemäß Anspruch 1.

4. Verwendung von Heterocyclyluracilen der Formel (I) gemäß Anspruch 1 zur Bekämpfung von Unkräutern.

5. Verfahren zur Bekämpfung von Unkräutern, **dadurch gekennzeichnet, dass** man Heterocyclyluracile der Formel (I) gemäß Anspruch 1 auf die Unkräuter und/oder deren Lebensraum ausbringt.

6. Verfahren zur Herstellung von herbiziden Mitteln, **dadurch gekennzeichnet, dass** man Heterocyclyluracile der Formel (I) gemäß Anspruch 1 mit Streckmitteln und/oder oberflächenaktiven Stoffen vermischt.

## Claims

1. Heterocyclyluracils of the formula 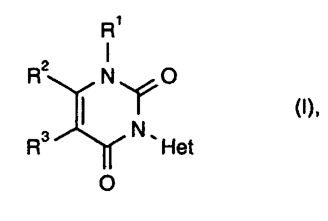 in which
R¹ represents hydrogen or represents optionally cyano-, halogen- or C₁-C₄-alkoxy-substituted C₁-C₄-alkyl,
R² represents formyl, hydroximinomethyl, cyano, carboxyl, alkoxycarbonyl, carbamoyl, thiocarbamoyl or represents optionally halogen-substituted C₁-C₄-alkyl,
R³ represents hydrogen, cyano, halogen or represents optionally halogen-substituted C₁-C₄-alkyl and
Het represents the radical of the formula 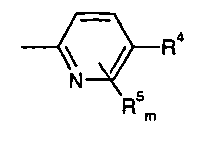 in which
R⁴ represents cyano or thiocarbamoyl,
R⁵ represents alkoxy having 1 to 6 carbon atoms, halogenoalkoxy having 1 to 6 carbon atoms and 1 to 5 halogen atoms, alkoxyalkoxy having 1 to 6 carbon atoms in each alkoxy moiety, alkylthio having 1 to 6 carbon atoms, halogenoalkylthio having 1 to 6 carbon atoms and 1 to 5 halogen atoms, alkylsulphonylamino having 1 to 6 carbon atoms in the alkyl moiety, halogenoalkylsulphonylamino having 1 to 5 halogen atoms and 1 to 6 carbon atoms in the halogenoalkyl moiety, N,N-bis-alkylsulphonylamino having 1 to 6 carbon atoms in each alkyl moiety, N,N-bis-halogenoalkylsulphonyl-amino having 1 to 5 halogen atoms and 1 to 6 carbon atoms in each halogenoalkyl moiety, N-alkyl-N-alkylsulphonyl-amino having 1 to 6 carbon atoms in the alkyl moiety and 1 to 6 carbon atoms in the alkylsulphonyl moiety, N-halogenoalkyl-N-halogenoalkylsulphonyl-amino having 1 to 5 halogen atoms and 1 to 6 carbon atoms in the halogenoalkyl moiety and 1 to 5 halogen atoms and 1 to 6 carbon atoms in the halogenoalkylsulphonyl moiety, N-alkylcarbonyl-N-alkylsulphonyl-amino having 1 to 6 carbon atoms in the alkyl moiety of the alkylcarbonyl group and 1 to 6 carbon atoms in the alkylsulphonyl moiety, N-halogenoalkylcarbonyl-N-halogenoalkylsulphonyl-amino having 1 to 5 halogen atoms and 1 to 6 carbon atoms in the halogenoalkyl moiety and 1 to 5 halogen atoms and 1 to 6 carbon atoms in the halogenoalkylsulphonyl moiety or represents N-alkylsulphonyl-N-phenylcarbonyl-amino having 1 to 6 carbon atoms in the alkylsulphonyl moiety and being optionally mono- to trisubstituted by identical or different substituents from the group consisting of halogen, alkyl having 1 to 4 carbon atoms, dialkylamino having 1 to 4 carbon atoms in each alkyl group and alkoxy having 1 to 4 carbon atoms, and
m represents integers from 0 to 3,
or
Het represents the radical of the formula 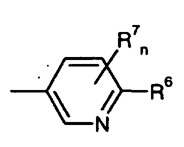 in which
R⁶ represents carbamoyl or thiocarbamoyl,
R⁷ represents alkoxy having 1 to 6 carbon atoms, halogenoalkoxy having 1 to 6 carbon atoms and 1 to 5 halogen atoms, alkoxyalkoxy having 1 to 6 carbon atoms in each alkoxy moiety, alkylthio having 1 to 6 carbon atoms, halogenoalkylthio having 1 to 6 carbon atoms and 1 to 5 halogen atoms, alkylsulphonylamino having 1 to 6 carbon atoms in the alkyl moiety, halogenoalkylsulphonylamino having 1 to 5 halogen atoms and 1 to 6 carbon atoms in the halogenoalkyl moiety, N,N-bis-alkylsulphonylamino having 1 to 6 carbon atoms in each alkyl moiety, N,N-bis-halogenoalkylsulphonyl-amino having 1 to 5 halogen atoms and 1 to 6 carbon atoms in each halogenoalkyl moiety, N-alkyl-N-alkylsulphonyl-amino having 1 to 6 carbon atoms in the alkyl moiety and 1 to 6 carbon atoms in the alkylsulphonyl moiety, N-halogenoalkyl-N-halogenoalkylsulphonyl-amino having 1 to 5 halogen atoms and 1 to 6 carbon atoms in the halogenoalkyl moiety and 1 to 5 halogen atoms and 1 to 6 carbon atoms in the halogenoalkylsulphonyl moiety, N-alkylcarbonyl-N-alkylsulphonyl-amino having 1 to 6 carbon atoms in the alkyl moiety of the alkylcarbonyl group and 1 to 6 carbon atoms in the alkylsulphonyl moiety, N-halogenoalkylcarbonyl-N-halogenoalkylsulphonyl-amino having 1 to 5 halogen atoms and 1 to 6 carbon atoms in the halogenoalkyl moiety and 1 to 5 halogen atoms and 1 to 6 carbon atoms in the halogenoalkylsulphonyl moiety or represents N-alkylsulphonyl-N-phenylcarbonyl-amino having 1 to 6 carbon atoms in the alkylsulphonyl moiety and being optionally mono- to trisubstituted by identical or different substituents from the group consisting of halogen, alkyl having 1 to 4 carbon atoms, dialkylamino having 1 to 4 carbon atoms in each alkyl group and alkoxy having 1 to 4 carbon atoms, and
n represents integers from 0 to 3,
or
Het represents the radical of the formula 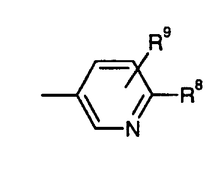 in which
R⁸ represents cyano,
R⁹ represents alkylsulphonylamino having 1 to 6 carbon atoms in the alkyl moiety, halogenoalkylsulphonylamino having 1 to 5 halogen atoms and 1 to 6 carbon atoms in the halogenoalkyl moiety, N,N-bis-alkylsulphonylamino having 1 to 6 carbon atoms in each alkyl moiety, N,N-bis-halogenoalkylsulphonylamino having 1 to 5 halogen atoms and 1 to 6 carbon atoms in each halogenoalkyl moiety, N-alkyl-N-alkylsulphonyl-amino having 1 to 6 carbon atoms in the alkyl moiety and 1 to 6 carbon atoms in the alkylsulphonyl moiety, N-halogenoalkyl-N-halogenoalkylsulphonyl-amino having 1 to 5 halogen atoms and 1 to 6 carbon atoms in the halogenoalkyl moiety and 1 to 5 halogen atoms and 1 to 6 carbon atoms in the halogenoalkylsulphonyl moiety, N-alkylcarbonyl-N-alkylsulphonyl-amino having 1 to 6 carbon atoms in the alkyl moiety of the alkylcarbonyl group and 1 to 6 carbon atoms in the alkylsulphonyl moiety, N-halogenoalkylcarbonyl-N-halogenoalkylsulphonyl-amino having 1 to 5 halogen atoms and 1 to 6 carbon atoms in the halogenoalkyl moiety and 1 to 5 halogen atoms and 1 to 6 carbon atoms in the halogenoalkylsulphonyl moiety or represents N-alkylsulphonyl-N-phenylcarbonyl-amino having 1 to 6 carbon atoms in the alkylsulphonyl moiety and being optionally mono- to trisubstituted by identical or different substituents from the group consisting of halogen, alkyl having 1 to 4 carbon atoms, dialkylamino having 1 to 4 carbon atoms in each alkyl group and alkoxy having 1 to 4 carbon atoms.

2. Process for preparing heterocyclyluracils of the formula (I) according to Claim 1, **characterized in that**
a) aminoalkenoic esters of the formula 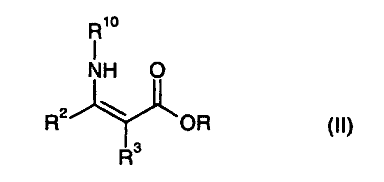 in which
R² and R³ are each as defined in Claim 1,
R¹⁰ represents hydrogen or alkyl having 1 to 4 carbon atoms and
R represents alkyl having from 1 to 4 carbon atoms, phenyl or benzyl
are either
α) reacted with heterocyclyl isocyanates of the formula
O=C=N-Het (III),
in which
Het is as defined in Claim 1,
or
β) reacted with heterocyclylcarbamates of the formula 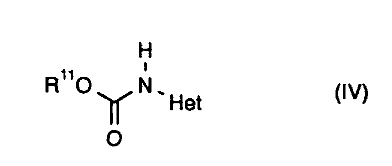
in which
Het is as defined in Claim 1 and
R¹¹ represents alkyl having from 1 to 4 carbon atoms, phenyl or benzyl,
in each case if appropriate in the presence of an acid binder and if appropriate in the presence of a diluent,
or
b) heterocyclyluracils of the formula 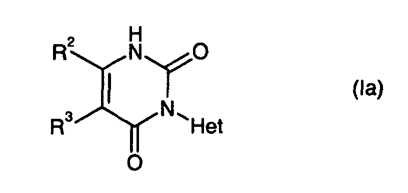
in which
R², R³ and Het are each as defined in Claim 1
are either
α) reacted with halogen compounds of the formula
R¹²-Hal (V)
in which
R¹² represents alkyl having 1 to 4 carbon atoms which is optionally substituted by cyano, halogen or alkoxy having 1 to 4 carbon atoms and
Hal represents chlorine, bromine or iodine,
or
β) reacted with dialkyl sulphates of the formula 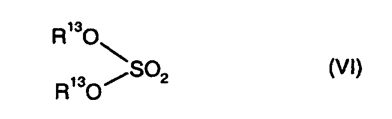
in which
R¹³ represents alkyl having 1 to 4 carbon atoms,
in each case if appropriate in the presence of an acid binder and if appropriate in the presence of a diluent.

3. Herbicidal compositions, **characterized in that** they comprise at least one heterocyclyluracil of the formula (I) according to Claim 1.

4. The use of heterocyclyluracils of the formula (I) according to Claim 1 for controlling weeds.

5. Method for controlling weeds, **characterized in that** heterocyclyluracils of the formula (I) according to Claim 1 are applied to the weeds and/or their habitat.

6. Process for preparing herbicidal compositions, **characterized in that** heterocyclyluracils of the formula (I) according to Claim 1 are mixed with extenders and/or surfactants.

## Revendications

1. Hétérocyclyluraciles de formule 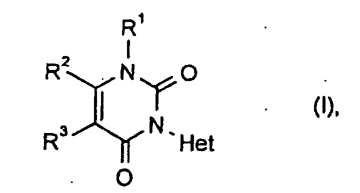 dans laquelle
R¹ représente l'hydrogène ou un reste alkyle en C₁ à C₄ portant éventuellement un substituant cyano, halogéno ou alkoxy en C₁ à C₄,
R² représente un reste formyle, hydroxyminométhyle, cyano, carboxy, alkoxycarbonyle, carbamoyle, thiocarbamoyle ou un reste alkyle en C₁ à C₄ éventuellement substitué par un halogène,
R³ représente l'hydrogène, un groupe cyano, un halogène ou un reste alkyle en C₁ à C₄ éventuellement substitué par un halogène, et
Het représente le reste de formule 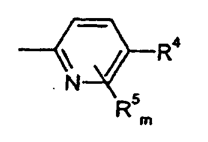 dans laquelle
R⁴ est un groupe cyano ou thiocarbamoyle,
R⁵ est un reste alkoxy ayant 1 à 6 atomes de carbone, un reste halogénalkoxy ayant 1 à 6 atomes de carbone et 1 à 5 atomes d'halogène, un reste alkoxy-alkoxy ayant 1 à 6 atomes de carbone dans chaque partie alkoxy, un reste alkylthio ayant 1 à 6 atomes de carbone, un reste halogénalkylthio ayant 1 à 6 atomes de carbone et 1 à 5 atomes d'halogène, un reste alkylsulfonylamino ayant 1 à 6 atomes de carbone dans la partie alkyle, un reste halogénalkylsulfonylamino ayant 1 à 5 atomes d'halogène et 1 à 6 atomes de carbone dans la partie halogénalkyle, un reste N,N-bis-alkylsulfonylamino ayant 1 à 6 atomes de carbone dans chaque partie alkyle, un reste N,N-bis-halogénalkylsulfonylamino ayant 1 à 5 atomes d'halogène et 1 à 6 atomes de carbone dans chaque partie halogénalkyle, un reste N-alkyl-N-alkylsulfonylamino ayant 1 à 6 atomes de carbone dans la partie alkyle et 1 à 6 atomes de carbone dans la partie alkylsulfonyle, un reste N-halogénalkyl-N-halogénalkylsulfonylamino ayant 1 à 5 atomes d'halogène et 1 à 6 atomes de carbone dans la partie halogénalkyle et 1 à 5 atomes d'halogène et 1 à 6 atomes de carbone dans la partie halogénalkyl-sulfonyle, un reste N-alkylcarbonyl-N-alkyl-sulfonylamino ayant 1 à 6 atomes de carbone dans la partie alkyle du groupe alkylcarbonyle et 1 à 6 atomes de carbone dans la partie alkylsulfonyle, un reste N-halogénalkylcarbonyl-N-halogénalkyl-sulfonylamino ayant 1 à 5 atomes d'halogène et 1 à 6 atomes de carbone dans la partie halogénalkyle et 1 à 5 atomes d'halogène et 1 à 6 atomes de carbone dans la partie halogénalkylsulfonyle ou un reste N-alkylsulfonyl-N-phényl-carbonylamino ayant 1 à 6 atomes de carbone dans la partie alkylsulfonyle, portant éventuellement 1 à 3 substituants, identiques ou différents, halogéno, alkyle ayant 1 à 4 atomes de carbone, dialkylamino ayant 1 à 4 atomes de carbone dans chaque groupe alkyle et/ou alkoxy ayant 1 à 4 atomes de carbone, et
m représente des nombres entiers de 0 à 3,
ou bien
Het représente le reste de formule 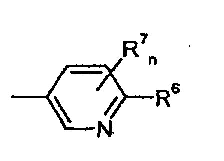 dans laquelle
R⁶ est un groupe carbamoyle ou thiocarbamoyle,
R⁷ est un reste alkoxy ayant 1 à 6 atomes de carbone, un reste halogénalkoxy ayant 1 à 6 atomes de carbone et 1 à 5 atomes d'halogène, un reste alkoxyalkoxy ayant 1 à 6 atomes de carbone dans chaque partie alkoxy, un reste alkylthio ayant 1 à 6 atomes de carbone, un reste halogénalkylthio ayant 1 à 6 atomes de carbone et 1 à 5 atomes d'halogène, un reste alkylsulfonylamino ayant 1 à 6 atomes de carbone dans la partie alkyle, un reste halogénalkylsulfonylamino ayant 1 à 5 atomes d'halogène et 1 à 6 atomes de carbone dans la partie halogénalkyle, un reste N,N-bis-alkylsulfonylamino ayant 1 à 6 atomes de carbone dans chaque partie alkyle, un reste N,N-bis-halogénalkylsulfonylamino ayant 1 à 5 atomes d'halogène et 1 à 6 atomes de carbone dans chaque partie halogénalkyle, un reste N-alkyl-N-alkylsulfonylamino ayant 1 à 6 atomes de carbone dans la partie alkyle et 1 à 6 atomes de carbone dans la partie alkylsulfonyle, un reste N-halogénalkyl-N-halogénalkylsulfonylamino ayant 1 à 5 atomes d'halogène et 1 à 6 atomes de carbone dans la partie halogénalkyle et 1 à 5 atomes d'halogène et 1 à 6 atomes de carbone dans la partie halogénalkylsulfonyle, un reste N-alkylcarbonyl-N-alkylsulfonylamino ayant 1 à 6 atomes de carbone dans la partie alkyle du groupe alkylcarbonyle et 1 à 6 atomes de carbone dans la partie alkylsulfonyle, un reste N-halogénalkylcarbonyl-N-halogénalkyl-sulfonylamino ayant 1 à 5 atomes d'halogène et 1 à 6 atomes de carbone dans la partie halogénalkyle et 1 à 5 atomes d'halogène et 1 à 6 atomes de carbone dans la partie halogénalkyl-sulfonyle, ou un reste N-alkylsulfonyl-N-phénylcarbonylamino ayant 1 à 6 atomes de carbone dans la partie alkylsulfonyle, portant éventuellement 1 à 3 substituants, identiques ou différents, halogéno, alkyle ayant 1 à 4 atomes de carbone, dialkylamino ayant 1 à 4 atomes de carbone dans chaque groupe alkyle et/ou alkoxy ayant 1 à 4 atomes de carbone, et
n représente les nombres entiers de 0 à 3,
ou bien
Het représente le reste de formule 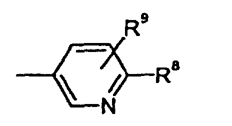 dans laquelle
R⁸ est un groupe cyano,
R⁹ est un reste alkylsulfonylamino ayant 1 à 6 atomes de carbone dans la partie alkyle, un reste halogénalkylsulfonylamino ayant 1 à 5 atomes d'halogène et 1 à 6 atomes de carbone dans la partie halogénalkyle, un reste N,N-bis-alkylsulfonylamino ayant 1 à 6 atomes de carbone dans chaque partie alkyle, un reste N,N-bis-halogénalkylsulfonylamino ayant 1 à 5 atomes d'halogène et 1 à 6 atomes de carbone dans chaque partie halogénalkyle, un reste N-alkyl-N-alkylsulfonylamino ayant 1 à 6 atomes de carbone dans la partie alkyle et 1 à 6 atomes de carbone dans la partie alkylsulfonyle, un reste N-halogénalkyl-N-halogénalkylsulfonyl-amino ayant 1 à 5 atomes d'halogène et 1 à 6 atomes de carbone dans la partie halogénalkyle et 1 à 5 atomes d'halogène et 1 à 6 atomes de carbone dans la partie halogénalkylsulfonyle, un reste N-alkylcarbonyl-N-alkylsulfonyl-amino ayant 1 à 6 atomes de carbone dans la partie alkyle du groupe alkylcarbonyle et 1 à 6 atomes de carbone dans la partie alkylsulfonyle, un reste N-halogénalkyl-carbonyl-N-halogénalkylsulfonylamino ayant 1 à 5 atomes d'halogène et 1 à 6 atomes de carbone dans la partie halogénalkyle et 1 à 5 atomes d'halogène et 1 à 6 atomes de carbone dans la partie halogénalkylsulfonyle, ou un reste N-alkylsulfonyl-N-phénylcarbonylamino ayant 1 à 6 atomes de carbone dans la partie alkylsulfonyle, portant éventuellement 1 à 3 substituants, identiques ou différents, halogéno, alkyle ayant 1 à 4 atomes de carbone, dialkylamino ayant 1 à 4 atomes de carbone dans chaque groupe alkyle et/ou alkoxy ayant 1 à 4 atomes de carbone.

2. Procédé de production d'hétérocyclyluraciles de formule (I) suivant la revendication 1, **caractérisé en ce que** :
a) on fait réagir des esters d'acides amino-alcénoiques de formule 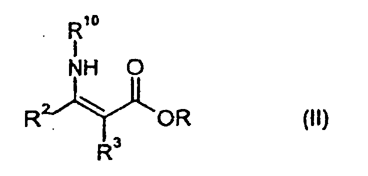 dans laquelle
R² et R³ ont les définitions indiquées dans la revendication 1,
R¹⁰ représente l'hydrogène ou un reste alkyle ayant 1 à 4 atomes de carbone,
et
R est un reste alkyle ayant 1 à 4 atomes de carbone, phényle ou benzyle,
soit
α) avec des hétérocyclylisocyanates de formule
O=C=N-Het (III),
dans laquelle
Het a la définition indiquée dans la revendication 1,
soit
β) avec des hétérocyclylcarbamates de formule 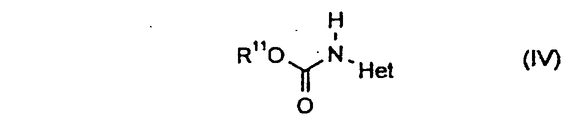
dans laquelle
Het a la définition indiquée dans la revendication 1, et
R¹¹ est un reste alkyle ayant 1 à 4 atomes de carbone, phényle ou benzyle,
dans chaque cas éventuellement en présence de l'accepteur d'acide et le cas échéant, en présence d'un diluant,
ou bien
b) on fait réagir des hétérocyclyluraciles de formule 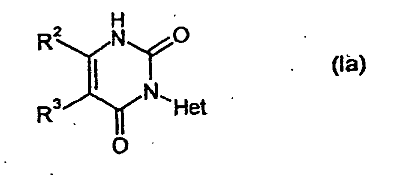
dans laquelle
R², R³ et Het ont les définitions indiquées dans la revendication 1,
soit
α) avec des composés halogénés de formule
R¹²-Hal (V)
dans laquelle
R¹² désigne un reste alkyle ayant 1 à 4 atomes de carbone, éventuellement substitué par un radical cyano, halogéno ou alkoxy ayant 1 à 4 atomes de carbone, et
Hal représente le chlore, le brome ou l'iode,
soit
β) avec des sulfates de diakyle de formule 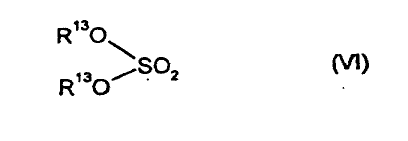 dans laquelle
R¹³ est un reste alkyle ayant 1 à 4 atomes de carbone,
dans chaque cas éventuellement en présence d'un accepteur d'acide et le cas échéant, en présence d'un diluant.

3. Compositions herbicides, **caractérisées par** une teneur en au moins un hétérocyclyluracile de formule (I) suivant la revendication 1.

4. Utilisation d'hétérocyclyluraciles de formule (I) suivant la revendication 1 pour combattre des mauvaises herbes.

5. Procédé de lutte contre des mauvaises herbes, **caractérisé en ce qu'**on épand des hétérocyclyluraciles de formule (I) suivant la revendication 1 sur les mauvaises herbes et/ou sur leur milieu.

6. Procédé de préparation de compositions herbicides, **caractérisé en ce qu'**on mélange des hétérocyclyluraciles de formule (I) suivant la revendication 1 avec des diluants et/ou des agents tensioactifs.
